# EUROPEAN PATENT SPECIFICATION

(11) **EP 1 203 086 B1**
(45) Date of publication and mention of the grant of the patent: **13.09.2006**
(21) Application number: 00946398.5
(22) Date of filing: 19.07.2000
(51) Int. Cl.: C12N 15/82, C12N 15/90, A01H 5/00

(54) **MITES-LIKE ELEMENT AND TRANSCRIPTIONAL ACTIVATION ELEMENT**
MITES-ÄHNLICHES ELEMENT UND AN TRANKRIPTIONSAKTIVIERUNG BETEILIGTES ELEMENT
ELEMENT SIMILAIRE DE MITE ET ELEMENT D'ACTIVATION DE TRANSCRIPTION

(30) Priority: 21.07.1999 JP 20631699; 21.07.1999 JP 20632099; 12.06.2000 JP 2000175825
(43) Date of publication of application: 08.05.2002
(73) Proprietor: SAN-EI GEN F.F.I., INC., Toyonaka, Osaka 561-8588 (JP); Ozeki, Yoshihiro, Tokyo 203-0011 (JP)
(72) Inventor: OZEKI, Yoshihiro, Higashikurume-shi, Tokyo 203-0011 (JP); OYANAGI, Mikiko, Fuchu-shi, Tokyo 183-0006 (JP); FUKUDA, Takashi, Sagamihara-shi, Kanagawa 229-1123 (JP); KODA, Takatoshi, San-Ei Gen F.F.I. Inc., Osaka 561-0828 (JP)
(74) Representative: HOFFMANN EITLE
(86) International application number: PCT/JP2000/004837
(87) International publication number: WO 2001/005986

(56) References cited:
- WO-A-98/22593
- BUREAU T E ET AL: "Stowaway: a new family of inverted repeat elements associated with the genes of both monocotyledonous and dicotyledonous plants" PLANT CELL,AMERICAN SOCIETY OF PLANT PHYSIOLOGISTS, ROCKVILLE, MD,US, vol. 6, no. 6, June 1994 (1994-06), pages 907-916, XP002156106 ISSN: 1040-4651 cited in the application
- B¹N¹DICTE CHARRIER ET AL.: "Bigfoot: a new family of MITE elements characterized from the Medicago genus" PLANT JOURNAL., vol. 18, no. 4, May 1999 (1999-05), pages 431-441, XP002157570 BLACKWELL SCIENTIFIC PUBLICATIONS, OXFORD., GB ISSN: 0960-7412
- ELENA CASACUBERTA ET AL.: "Presence of miniature inverted-repeat transposable elements (MITEs) in the genome of Arabidopsis thaliana: characterisation of the Emigrant family of elements" PLANT JOURNAL., vol. 16, no. 1, October 1998 (1998-10), pages 79-85, XP002156103 BLACKWELL SCIENTIFIC PUBLICATIONS, OXFORD., GB ISSN: 0960-7412
- SHIN TAKEDA ET AL.: "Transcriptional activation of the tobacco retrotransposon Tto1 by wounding and methyl jasmonate" PLANT MOLECULAR BIOLOGY, vol. 36, 1998, pages 365-376, XP002165578

## Description

### TECHNICAL FIELD

The present invention relates to novel, plant-derived transposable elements and, more particularly, to a MITE (miniature inverted-repeat transposable element)-like sequence. More specifically, the present invention relates to novel transposable elements isolated from carrot (*Daucus carota*).

The invention further relates to novel transcriptional activation elements containing the transposable elements. More specifically, it relates to a transcriptional activation element which, when inserted in a gene, is capable of promoting the transcription of a gene around or in the vicinity of the site of insertion thereof or suppressing the transcription of said gene from being inactivated. The invention still further relates to a transgenic plant harboring said transcriptional activation element.

### BACKGROUND ART

Transposable elements are found in the genomes of almost all living organisms, without distinction between prokaryotes and eukaryotes, or between animals and plants. It is known that these transposable elements can move from a genomic gene to another and inactivate the genomic gene upstream or downstream from the sequence in which such an element has been inserted. Further, it has been revealed that, in addition to such actions, transposable elements cause various types of genomic reorganization (deletion, inversion, duplication, etc.) and it has been reported that the above fact leads to genome plasticity, which is important for the evolution of living organisms and, in particular, that transposable elements play an important role in the evolution of living organisms for environmental adaptation in response to genomic stresses (McClintock, Science **26**: 792-801 (1984); Arber et al., FEMS Microb. Ecol., 15: 5-14 (1994)).

Transposable elements are roughly classified into three types: DNA type (transposons and insertion sequences), RNA type (retrotransposons) (Berg et al. (ed.), Mobile DNA (1989)), and miniature inverted-repeat transposable elements (MITEs) belonging to neither of the above two types (Wessler et al., Curr. Opin. Genet. Dev. 5: 914-821 (1995)).

As for DNA type and RNA type transposable elements among them, their actual transposition in the genome has been established whereas, for MITEs, no reports have so far been made about evidences of their transposition in the genome in spite of their being very similar to DNA type ones.

Most of MITEs have been found out, by computer retrieval, from databases of nucleotide sequences of genomic genes of various living organisms as being elucidated by genome projects currently in progress. The first discovery was the discovery in 1992 of the *T*o*urist* family by Bureau et al. (Bureau et al. , Plant Cell 4: 1283-1294 (1992)). Since then, various MITEs have been found in plant genomes and in insect and animal genomes.

Generally, MITEs are defined as having such characteristics as (1) their having a perfect or imperfect inverted repeat sequence at each of both the 5'- and 3'-terminal regions (similar in this respect to DNA type transposable elements), (2) occurrence of a target duplication-like sequence, of a sequence consisting of two or more nuclueotides, like the one formed upon insertion of a DNA type transposable element into a genomic DNA, on both the terminal sides of the inverted repeat sequences, and (3) their generally having a size of shorter than 2 kb (Wessler et al. , Curr. Opin. Genet. Dev. 5: 914-821 (1995)).

As those MITEs which have an open reading frame coding for a transposase between both the terminal inverted repeat sequences, like DNA type transposable elements, there are known only MITEs of the IS630-Tc1/Mariner family found in molds and animals (Kachroo et al. , Mol. Gen. Genet. 245: 339-348 (1994); Smit et al., Proo. Natl. Acad. Sci. USA 93: 1443-1448 (1996)). As far as plant-derived MITEs are concerned, however, no such transposase-enooding open reading frame has been discovered. Therefore, many points remain unelucidated concerning the mechanisms of transposition of plant MITEs and concerning their actions.

By the way, the gene transfer experiments so far made in gene manipulation in higher living organisms are mostly of the nuclear genome insertion type. This is because higher living organisms have no equivalent to the plasmid in prokaryotes.

For such gene transfer by insertion into the nuclear genome, there are available physical methods comprising introducing a vector coupled with a desired gene for insertion into the nuclear genome of a higher living organism by the particle gun, lipofection or electroporation technique, and biological methods comprising introducing said vectors harboring the genes once into a virus or microorganism and then introducing the same into the nuclear genome of a higher living organism by taking advantage of the DNA transfer/insertion system which said virus or microorganism has.

These physical and biological methods, however, each has a problem in that the expression activity of the gene inserted in a higher living organism varies from an individual to another, hence is not constant. In many instances, this is caused by silencing due to the position effect (Peach et al., Plant Mol. Biol. 17: 46-60 (1991)). Such the position effect is a phenomenon found in yeasts and many other eukaryotes and it is known that while there is no change in the nucleotide sequence of the inserted gene itself, the expression activity thereof varies markedly depending on the site of insertion of said gene on the chromosome and, in certain oases, the expression of the gene is completely inactivated (gene silencing) (Molecular Biology of the Cell, 3rd edition, 434-435 (1995)).

Supposed as the causes of such phenomenon of the gene silencing are the fact that all genes in the nuclear genome of a higher living organism are not uniformly transcribed and the fact that active sites where the gene is actively transcribed and the cryptic sites where transcription of the gene is silenced at all are intermingled in the nuclear genome. It is known that a gene inserted in a cryptic site cannot be transcribed at all due to complete failure of proteins necessary for transcription to approach the gene or due to a change in nucleosome structure or heterochromatinization as resulting from methylation of the genomic DNA in this site, with the result that inactivation of gene expression (gene silencing) occurs (Ng et al. , Curr. Opin. Genet. Dev. , 9: 158-163 (1999); Matzke et al., Curr. Opin. Plant Biol. 1: 142-148 (1999)).

It is further known that, even in the same organism, this gene silencing depends on the state of cell differentiation, as seen with X chromosome of manmal. Furthermore, it is known that once gene silencing is caused by a presently unknown mechanism, it is inherited by the offspring of the next generation resulting from mating (Molecular Biology of the Cell, 3rd edition, 434-453 (1995)).

Meanwhile, when, in gene manipulation, a desired gene (foreign gene) is introduced into higher animal cells by a physical or biological method (other than the homologous recombination method used for gene knockout), the site of insertion of the gene in the cell nuclear genome differs among cells and it is substantially impossible to control it artificially. Thus, introduction of a foreign gene into cells of a higher living organism results in formation, in an uncertain manner, of cells containing the foreign gene in an active site of the nuclear genome and cells containing the gene in a cryptic site of the genome.

In the case of plant, it is known that a foreign gene, once introduced in a cryptic site, undergoes the influence of the cryptic site, with the result that the expression of the foreign gene markedly diminishes or becomes null and it is also known that even when a foreign gene is inserted in an active site, the expression of the foreign gene becomes unstabilized and diminishes due to gene silencing as the growth progresses by repetitions of cell division (Peach et al., Plant Mol. Biol. 17: 46-60 (1991)).

A main presumable cause of inactivation of the expression of a foreign gene is the structure of the nuclear DNA in the chromosome. An element participating in the structure of the nuclear DNA is a MAR (matrix attachment region; also called SAR (scaffold attachment region) sequence. This was found as a sequence anchoring a nuclear genomic DNA to a nuclear matrix. In animals, it was shown that when a MAR-containing chicken A element is ligated to a foreign gene to be inserted followed by gene transfer, the expression of the gene inserted increased (Stief et al. , Nature 341; 343-345 (1989)). Later studies, however, revealed that the MAR contributes to an increase in gene expression efficiency but it, when alone, cannot counteract the position effect (Bonifer et al., Nculeic Acids Res. 22: 4202-4210 (1994); Poljak et al., Nucleic Acids Res. 22: 4386-4394 (1994)). In plants as well, studies have been made to investigate the effects of MARs using transformants. However, any reproducible results have not been obtained as yet. It is not considered that a MAR alone can counteract the position effect (Meshi: Shokubutsu no Genome Science (Genome Science of Plants), 153-160 (1996), published by Shujunsha).

Currently, genetically modified plants have been developed as genetically modified foodstuffs. However, the most important problem to be solved in developing them is the phenomenon of gene silencing which causes Inactivation of the expression of foreign genes, as mentioned hereinabove. In developing genetically modified plants, it is considered necessary, for avoiding the phenomenon of gene silencing, to select, among a very large number of plant individuals, plant individuals having a foreign gene inserted at a site where gene silencing is caused as scarcely as possible and, further, to select plant individuals in which gene silencing will not occur even after numerous generation for many years. For such selection, it is necessary to raise a large number of plant individuals and repeat over a number of generations and, therefore, not only much labor and time are required but also a vast area of land is required, namely a soil area problem arises.

Therefore, as another strategy of avoiding gene silencing, it is an urgent and most important problem in genetic engineering of plants to develop a method of suppressing the position effect-due gene silencing or activating the transcription of an inserted foreign gene and, more specifically, develop the so-called "transcriptional activation element" having either of these effects.

### DISCLOSURE OF INVENTION

As mentioned hereinabove, many points remain unelucidated as to what functions plant MITEs have. As for the functions of MITEs, the possibility is presumable of their contributing to the activation of gene expression based on the finding obtained by studies so far made that MITEs are found frequently in regions upstream of promoters of various genes (Wessler et al., Curr. Opin. Genet. Dev. 5: 914-821 (1995)). Further, since a number of MITEs are found in the vicinity of a matric attachment region (MAR) (Avramova et al., Nucleic Acids Res. 26: 761-767 (1998)), it is also presumable that they have an important connection with the structure of the genome.

Thus, MITEs are very interesting transposable elements (insertion elements) from the scientific viewpoint in elucidating the relation between the genomic structure and the gene expression, which has not yet been exhaustively investigated. In view of the possibility of MITES, when incorporated in a genomic structure after transposition between genomic genes, changing the genomic structure and, as a result, controlling the gene expression, their utility as factors stabilizing, or preventing inactivation of , the gene expression activity on the occasion of foreign gene introduction can also be expected.

Thus, in a first aspect, the present invention has for its object to provide novel, plant-derived MITE-like elements, which have scientific and industrial utility, as mentioned above.

Specifically, the invention relates, first of all, to the novel MITE-like elements mentioned below under 1 (hereinafter, such MITE-like elements are sometimes referred to also as "IS2 elements" for convenience):
1. A MITE-like element consisting of the following DNA (a) or (b) :
   (a) a DNA having a nucleotide sequence shown under SEQ ID NO:1
   (b) a DNA capable of hybridizing with a DNA having a complementary sequence to the above nucleotide sequence (a) under stringent conditions and capable of causing duplication of the target sequence: (A)nG(A)n [n being an integer of not less than 1] at the site of insertion thereof in a genomic gene.

   In its second aspect, the present invention has for its object to provide the so-called "transcriptional activation factor" capable of suppressing the gene inactivation phenomenon called gene silencing due to a position effect or capable of activating the transcription of a gene located in the vicinity or marginal region thereof.
   Specifically, the invention relates to the following transcriptional activation factors mentioned below under 2 to 6:
2. A transcriptional activation element characterized by containing at least one MITE-like element as shown in 1 as a transposable element.
3. A transcriptional activation element as shown in 2, wherein the transposable element comprises at least one MITE-like element consisting of the following DNA (a) or (b) :
   (a) a DNA having the nucleotide sequence shown under SEQ ID No: 1:
   (b) a DNA capable of hybridizing with a DNA having a complementary sequence to the above nucleotide sequence (a) under stringent conditions and capable of causing duplication of (A)nG(A)n [n being an integer of not less than 1] at the site of insertion thereof in a genomic gene.
4. A transcriptional activation element as shown in 2, wherein the transposable element is a tandem coupling product from a MITE-like element consisting of the following DNA (a) or (b):
   (a) a DNA having the nucleotide sequence shown under SEQ ID NO:1:
   (b) a DNA capable of hybridizing with a DNA having a complementary sequence to the above nucleotide sequence (a) under stringent conditions and capable of causing duplication of (A) nG (A) n [n being an integer of not less than 1] at the site of insertion thereof in a genomic gene, and a MITE-like element consisting of the following DNA (c) or (d) :
   (c) a DNA having the nucleotide sequence shown under SEQ ID NO: 2
   (d) a DNA capable of hybridizing with a DNA having a complementary sequence to the above nucleotide sequence (c) under stringent conditions and capable of causing duplication of TA at the site of insertion thereof in a genomic gene.
5. A transcriptional activation element comprising a DNA having the nucleotide sequence shown under SEQ ID N0:3.
6. A transcriptional activation element comprising a DNA having the nucleotide sequence shown under SEQ ID NO:14.
   The present invention further relates to a cassette for expression of a gene introduced which comprises the transcriptional activation element mentioned above. Specifically, there may be mentioned the cassettes mentioned below under 7 to 9 :
7. A transgene expression cassette which comprises the transcriptional activation element of any of 2 to 6, and a DNA sequence operatively joined to said element.
8. A transgene expression cassette as shown in 7, wherein the DNA sequence operatively joined to the transcriptional activation element comprises a promoter and/or a terminator.
9. A transgene expression cassette as shown in 8, which further comprises, as the DNA sequence operatively joined to the transcriptional activation element a desired transgene sequence to be expressed.

The present invention further relates to a plasmid which contains the transcriptional activation element mentioned hereinabove (as an introduced gene expression cassette, for instance) and to a transgenic plant harboring the transcriptional activation element introduced therein by utilizing such plasmid.

### BRIEF DESCRIPTION OF THE DRAWINGS

Fig. 1 is a representation of the structure of an IS2 element, which is a MITE-like element according to the invention.
Fig. 2 (comparative only) is a representation of the structure of an IS1 element, and of its terminal inverted repeat sequences and its inserted duplicate sequence (TA in the underlined parts).
Fig. 3 is a schematic representation of a method of constructing gDCPAL3-pro/SK.
Fig. 4 is a comparative representation of the structures of the carrot PAL genes gDCPAL3 and gDCPAL4.
Fig. 5 is a (comparative) representation of the results of comparison between the nucleotide sequence of a MITE-like element (IS1 element) and the nucleotide sequences of the so far known *Stowaway* family (Bureau and Wessler, Plant Cell, 6: 907-917 (1994). Sequences indicated by white letters on a black background are terminal inverted repeat sequences showing homology.
Fig. 6 is a representation of the imperfect inverted repeat sequences and target duplication sequence (AAAAGAAAA in the underlined parts) terminally found in an IS2 element, which is a MITE-like element of the invention.
Fig. 7 (comparative only) is a schematic representation of a method of constructing IS1-35S/SK.
Fig. 8 is a schematic representation of a method of constructing IS2-35S/SK.
Fig. 9 is a schematic representation of a method of constructing IS12-35S/SK.
Fig. 10 is a schematic representation of a method of constructing MU3-35S/SK.
Fig. 11 is a schematic representation of a method of constructing pIS1-35S/AB35S, pIS2-35S/AB35S, pIS12-35S/AB35S and pMU3-35S/AB35S.
Fig. 12 is a representation of the results of Example 3 (1) in which a comparison was made among the numbers of regenerated calli, on selection media containing kanamycin, from cultured tobacco BY-2 cells transformed by introduction of the constructs pIS1-35S/AB35S (IS1), pIS2-35S/AB35S (IS2), pIS12-35S/AB35S (IS12) and pAB35S (35S) (control). In the figure, the upper and lower graphs show the results obtained by using selection media containing 100 *µ*g/ml and 300 *µ* g/ml of kanamycin, respectively.
Fig. 13 is a representation of the results of Example 3 (2) in which a comparison was made between the GUS activity of tobacco calli (control) resulting from introduction of pAB35S (35S) (left graph) and the GUS activity of tobacco calli resulting from introduction of pIS12-35S/AB35S (IS12) (right graph).

### BEST MODES FOR CARRYING OUT THE INVENTION

### I. Novel MITE-like elements

The known MITESs so far discovered include not only the *T*o*urist* and S*towaway* families mentioned above but also the families Cast-away. *Crackle, Emigrant, Explorer, Ditto, Gaijin, Krispie,* *Pop*, *Snap, Wanderer, Wujin, Wukong,* Wuneng and *Xbr*. While these MITEs are structurally characterized by all having a perfect or imperfect invereted repeat sequence in the terminal region, the inverted repeat sequences quite differ in nucleotide sequence and length among the MITEs. Further, the target duplication sequence in the MITE is TAA for *Tourist*, TA for *Stowaway,* TAA for *Castaway,* GTTGATAT for *Crackle,* TTA for *Ditto,* TA for *Emigrant,* ATT or TAG or TGA or GGT or GTT or GAA for *Gaijin,* TTGAAC for *Krispie,* AAAACAAA or AAAAAAAA for *Pop*, TTTTTTT for *Snap,* TTA or TAA for *Wanderer,* TA or CATA for *Wujin,* TATA or TACA for *Wukong,* TTAA or TTAT for *Wuneng,* and TTAA for *Xbr* (any definite target duplication sequence has not been found for *Explorer*) (Bureau et al. , Proc. Natl. Acad. Sci. USA, 93: 8524-8529 (1996); Casacuberta et al. , Plant J., 16: 79-85 (1998); Song et al., Mol. Gen. Genet., 258: 449-456 (1998) ; Tu, Proc. Natl. Acad. Sci. USA, 94: 7475-7480 (1997); Unsal and Morgan, J. Mol. Biol., 248: 812-823 (1995)).

On the other hand, the IS2 element, an embodiment of the above-mentioned novel MITE-like element of the present invention has, as the target duplication sequence, (A)nG(A)n [n being an integer not less than 1], which is not found in any of the so far known MITEs (insertion elements). In this respect, the IS2 element can be said to be an insertion element belonging to a novel family different from any of the so far known families.

As for the IS1 element, it has TA as the target duplication sequence and thus can be said to be a novel MITE-like element belonging to the known family *Stowaway* (Bureau, T. E. and Wessler, S. R., *Stowaway:* a new family of inverted repeat elements associated with the genes of both monocotyledonous and dicotyledonous plants. Plant Cell, 6: 907-16 (1994)).

In the following, these IS2 and IS1 (comparative) elements are described.

### 1. IS2 element

The IS2 element is characterized by causing target duplication of (A)nG(A)n in the genomic gene at the site of insertion. The number n may be any integer not less than 1 . While it is not particularly restricted, it is specifically, for example 2 to 6, preferably 3 to 5, more preferably 4.

More specifically, the MITE-like element of the present invention is a DNA having a size of not more than about 2 kb, preferably about 0.2 to 2 kb, has repeat sequences reverse in direction to each other (terminal inverted repeat sequences) in the 5' and 3' terminal regions thereof.

From such viewpoint, the IS2 element of the present invention meets the requirements concerning the above-mentioned three characteristics of MITEs (Wessler et al. Curr. Opin. Genet. Dev. 5.: 914-821 (1995)), namely (1) that they have a perfect or imperfect inverted repeat sequence at each of the 5' and 3' termini, (2) that a repeat sequence comprising two or more base pairs is found as a target duplicate sequence in the same direction on both sides of the inverted repeat sequence of the gene insertion site and (3) that their size is not more than 2 kb, hence can be identified as a transposable element (insertion element, MITE-like element) having a MITE-like sequence.

The IS2 element of the present invention is structurally characterized by containing, in the nucleotide sequence thereof, at least one nucleotide sequence represented by the formula (1): XttgcaaY (wherein X represents g or t and Y represents a or c) (SEQ ID NO: 4 to 7) or the formula (2): Zatgcaa (wherein Z represents t or a) (SEQ ID NO: 8 to 9) in a continuously or discontinuously repeated manner.

The positioning and number of such repeat sequences are not particularly restricted but they may be contained in the terminal inverted repeat sequences occurring in both the terminal regions of the IS2 element or in an intermediate region occurring between said terminal inverted repeated sequences.

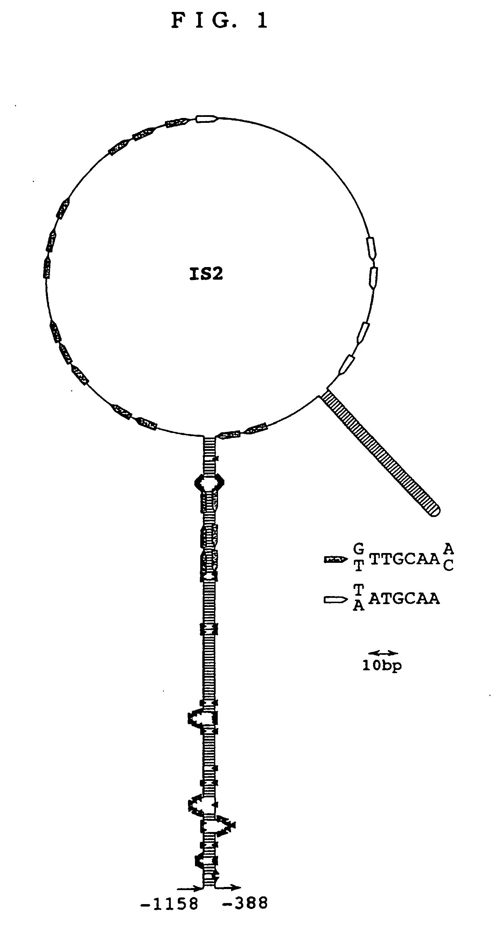

The IS2 element of the present invention specifically includes the ones which contain a plurality of repeat sequences represented by the above formula (1) and (2) in the intermediate region between the terminal inverted repeat sequences and a plurality of repeat sequences represented by the formula (1) in the terminal inverted repeat sequences, as shown in Fig. 1.

The terminal inverted repeat sequences which the IS2 element of the present invention has need not be strictly complementary to each other but the only requirement is that the 5' and 3' terminal regions can hybridize with each other under stringent conditions and, as a result, the IS2 element can have such a stem structure as shown in Fig. 1. In this sense, the IS2 element of the present invention Includes not only those having perfect inverted repeat sequences as the terminal inverted repeat sequences but also those having imperfect inverted repeat sequences as the terminal inverted repeat sequences.

As specific examples of the IS2 element according to the present invention, there may be mentioned the ones which have, as the terminal inverted repeat sequences, the nucleotide sequence shown under SEQ ID NO : 10 in the 5' terminal region and the nucleotide sequence shown under SEQ ID NO: 11 in the 3' terminal region. As a more specific example of the IS2 element, there may be mentioned the one having the nucleotide sequence shown under SEQ ID NO: 1 The IS2 element may have one or more nucleotides substituted, added or deleted in the terminal inverted repeat sequences or in the sequence occurring between said repeat sequences if the resulting modifications remain functional equivalents substantially having the function or activity of the IS2 element itself. The MITE-like element of the present invention includes such functional equivalents as well.

As preferred functional equivalents, there may be mentioned the ones substantially having the function or activity of the IS2 element having the nucleotide sequence shown under SEQ ID NO: 1 and causing target duplication of (A)nG(A)n [n being an integer not less than 1] at the site of insertion and capable of hybridizing with the above IS2 element under stringent conditions. As "stringent conditions", there may be mentioned the conditions in 1 x SSC plus 0.1% (w/w) SDS at 50**°** C or above over a period of 1 hour. As the functional equivalents, there may be mentioned more specifically the ones not less than 70%, preferably not less than 85%, more preferably not less than 90%, still more preferably not less than 95% homologous in nucleotide sequence with the IS2 element shown under SEQ ID NO 1.

### 2. IS1 element (comparative)

The IS1 element brings about target duplication of TA at the site of genomic gene insertion and is characterized by having, as the terminal inverted repeat sequences, the nucleotide sequence shown under SEQ ID NO: 12 in the 5' terminal region and the nucleotide sequence shown under SEQ ID NO: 13 in the 3' terminal region. The IS1 element is specifically a DNA having a size of not more than about 1 kb, preferably about 100 bp to 500 bp.

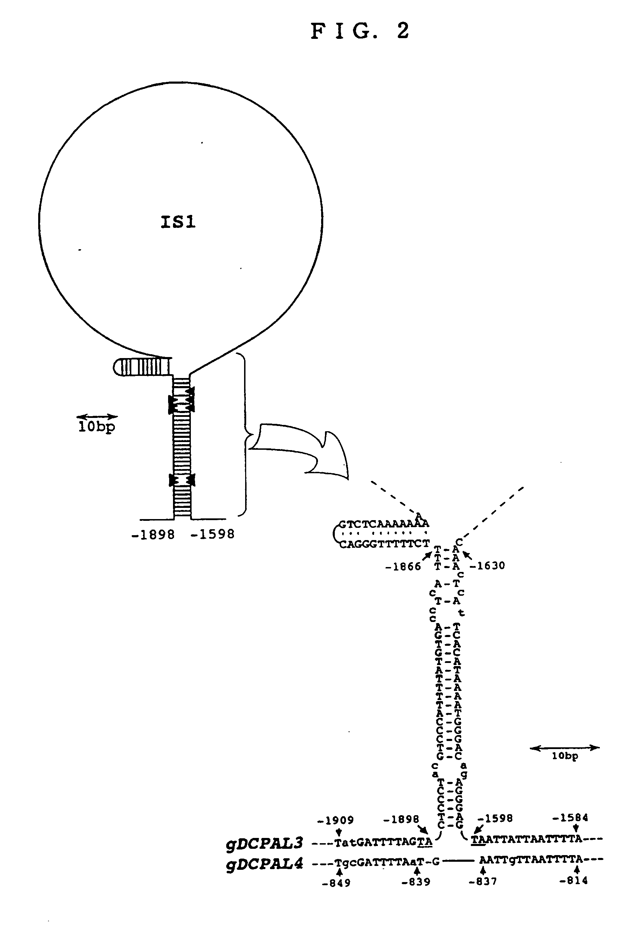

As the IS1 element there may specifically be mentioned the one having the structure shown in Fig. 2. More specifically, there may be mentioned the one having the nucleotide sequence shown under SEQ ID NO:2. The MITE-like element having such nucleotide sequence may have one or more nucleotides substituted, added or deleted in the terminal inverted repeat sequences or in the sequence occurring between these repeat sequences of the 5' and 3' terminal regions if the resulting modifications remain functional equivalents substantially having the function or activity of the MITE-like element itself.

Preferred as functional equivalents are those which substantially have the function or activity of the MITE-like element (IS1 element) having the nucleotide sequence shown under SEQ ID NO: 2 and which are at least 85%, preferably at least 90%, more preferably at least 95% homologous in nucleotide sequence with said IS1 element.

The MITE-like elements (IS2 and IS1 elements) described hereinabove all have been discovered from the carrot genome, more specifically from the carrot phenylalanine ammonia-lyase gene, as mentioned later herein, and can be isolated and recovered as described later herein in the example section. The MITE-like element of the present invention is not limited in origin provided that it has the structure and characteristics mentioned hereinabove.

It is generally pointed out that transposable elements, as self-mechanisms of automodification of the plant genome itself, might possibly contribute markedly to evolution of the organism concerned and to environmental adaptation.

As regards the MITE-like element of the present invention, it is unknown as to the mechanisms by which it functions in relation to the self-mechanisms of automodification of plant genomes. However, unlike the case of retrotransposons (McDonald, BioScience 40: 183-191 (1990), such a fact that it has an enhancer element therewithin and a gene promoter is activated by insertion of said enhancer element as resulting from transposition of said element is not found.

Therefore, the MITE-like element of the present invention can be considered to highly possibly cause changes in genomic structure upon insertion thereof in a plant genome and thereby contribute to dynamic changes of the genomic structure, such as changes in unwindability of the genomic DNA or in nuoleosome structure, by the mechanisms quite different from those of the so far known enhancer elements.

With the MITE-like element of the present invention, it becomes possible, by utilizing the above property, to control the expression of a gene located in the vicinity of said element by a technique different from the so far known techniques. It is generally pointed out that when a foreign gene is inserted in a cryptic site of a genomic DNA, the expression thereof is suppressed or inactivated. Therefore, it is considered possible, by utilizing the NITE-llke element of the present invention and based on the above properties, to invigorate or activate the reduced or suppressed ability to be expressed of a foreign gene introduced by transgenic technique. Accordingly, the MITE-like element of the present invention is useful in constructing a transgene expression cassette and a plasmid containing said cassette in stably creating genetically engineered plants and also useful in stably creating genetically engineered organisms capable of expression of the transgene by utilizing said cassette and plasmid.

### II. Transcriptional activation element

The present invention further relates to a transcriptional activation element.

The transcriptional activation element so referred to herein includes a element capable of promoting the transcription of a group of genes located in the vicinity or marginal region of said element as well as a element capable of inhibiting suppression of a desired foreign gene introduced in a genomic DNA or the like from being inactivated by gene silencing due to the position effect. The so-far known elements (factors) in charge of transcriptional activation each activates the transcription of a specific gene by occurring, as an enhancer, in the vicinity of the promoter of said gene and cis-acting directly on said promoter. On the contrary, the transcriptional activation element of the present invention promotes the transcription of a single gene or a group of a plurality of genes located in the vicinity or marginal region thereof, irrespective of position relative to the promoter and, further, includes those substantially promoting the transcription by suppressing the inherent phenomenon of transcription inactivation, hence conceptually includes a broader range of factors as compared with the prior art concept of transcriptional activation element.

The transcriptional activation element of the present invention is characterized by containing at least one transposable element.

The transposable element so referred to herein includes all of the above-mentioned DNA type and RNA type ones and MITEs. Thus, the transoriptional activation element of the present invention includes those containing at least one of these as a transposable element, regardless of whether it is derived from the same species or a different species .

A preferred transcriptional activation element contains a MITE (s) as the transposable element.

While a MITE is defined, as mentioned above, as an element having such characteristics as (1) having a perfect or imperfect inverted repeat sequence in each of both 5' and 3' terminal regions (similar in this respect to DNA type transposable elements), (2) having, on both sides of the inverted repeat sequence, a target duplication sequence comprising repeat sequences arranged in the same direction and comprising two or more base pairs, like the ones formed upon insertion of a DNA type transposable element in a genomic DNA and (3) having a size generally of shorter than 2 kb (Wessler et al., Curr. Opin. Genet. Dev. 5: 914-821 (1995)), any MITE belonging to such category of definition can be used as the transposable element in carrying out the present invention. As MITEs particularly suited among others, there may be mentioned the above-mentioned novel MITE-like elements of the present invention, namely the "IS2 element", and functional equivalents thereto.

Thus, the transcriptional activation element of the present invention preferably contains at least one nucleotide sequence selected from among said IS2 element, and functional equivalents thereto.

More specifically, the transcriptional activation element of the present invention includes, among others, (1) the one having the nucleotide sequence of the IS2 element or a functional equivalent thereof, (2) the one having a nucleotide sequence resulting from tandem joining of the nucleotide sequence of the IS1 element or a functional equivalent thereof and the nucleotide sequence of the IS2 element or a functional equivalent thereof (the order of the IS1 element and IS2 element being arbitrary) and (3) the one having a nucleotide resulting from joining of the nucleotide sequence of the IS1 element (or IS2 element) or a functional equivalent thereto and the nucleotide sequence of the IS2 element (or IS1 element) or a functional equivalent thereto via an arbitrary nucleotide sequence. Preferred examples of the transcriptional activation factor, there may be mentioned (i) the IS2 element or a functional equivalent thereto and (ii) the product of tandem joining of the IS1 element (or IS2 element) or a functional equivalent thereto and the IS2 element (or IS1 element) or a functional equivalent thereto. As a specific example of the latter (ii), there may be mentioned the one having the nucleotide sequence shown under SEQ ID NO:3.

Referring to the transcriptional activation element mentioned above under (3), the intervening nucleotide sequence between the IS1 element and IS2 element (the order being arbitrary) is not particularly restricted but may be any nucleotide sequence on condition that the effects of the invention are not counteracted. As a specific, but nonlimitative, example, there may be mentioned one derived from the carrot PAL promoter sequence. Generally, such nucleotide sequence can have a size of 5 to 1,000 bp, preferably 300 to 500 bp. As a transcriptional activation element of such mode of embodiment, there may be specifically mentioned the one having the nucleotide sequence shown under SEQ ID NO:14.

The transcriptional activation element of the present invention can be operatively joined to a desired gene sequence to be introduced into a plant body (transgene sequence (inclusive of foreign gene sequence)) and, further, the transcriptional activation element joined to said transgene sequence can be operatively joined to a functional DNA sequence or sequences, such as a promoter functional in a plant and/or a terminator functional in a plant.

The expression "operatively joined" as used herein means that the transcriptional activation element is located at a position sufficiently close to the above-mentioned transgene sequence or various functional DNA sequences to exert its influence on these sequences, irrespective of insertion site and direction.

The transgene to be used in the practice of the present invention includes those DNAs which are desired to be expressed in plants, whether homologous or heterologous to said plants. Such transgene includes, but is not limited to, genes coding for *β* -glucuronidase; antibiotic resistance genes; genes coding for insecticidal and bactericidal proteinaceous toxins; genes for antipathogenic compounds; genes synthesizing hypersensitivity compounds, such as peroxidases, gluoanases, chitinases, and phytoalexins; agrochemical, herbicide and microbicide resistance genes; genes synthesizing plant enzymes (e.g. enzymes connected with the contents and qualities of proteins, starch, saccharides and fats) and genes for regulatory factors therefor; genes related to plant enzyme inhibitors, such as protease and amylase inhibitors; genes involved in plant hormone synthesis; genes involved in insect hormone and pheromone synthesis; genes involved in the synthesis of medicinal and nutritional compounds, such as β-carotene and vitamins; and antisense transcripts interfering with nucleotide sequences occurring in plants (Transgenic Plant, vol. 1, Academic Press, 1993).

The present invention further relates to a gene expression cassette suited for application to plants, which comprises the above transcriptional activation element and a DNA sequence or sequences operatively joined thereto. The "gene expression cassette" so referred to herein means a plasmid to be used for introduction into plants as well as a subfragment thereof.

As the DNA sequence operatively joined to the transcriptional activation element, there may be mentioned functional DNA sequences such as promoter and terminator. Said DNA sequence can include any of the above-mentioned transgene sequences.

The promoter, so referred to herein, means a DNA sequence which, when the structural gene for a desired protein is joined thereto downstream from said promoter, can regulate the expression of said protein via transcription followed by translation in plant cells, and it includes all promoters functional in plants and used in the relevant field of art for the transformation of plants. A number of promoters have so far been used in transforming plants, including, for example, the promoters isolated from Agrobacterium tumefacians, namely the octopine synthase (ocs) promoter (L. Comai et al., 1985; C. Waldron et al., 1985), mannopine synthase (mas) promoter and nopaline synthase (nos) promoter. The cauliflower mosaic virus 35S promoter, which is generally used in the transformation, can adequately be used in practicing the present invention. Modifications of the 35S promoter, for example the two parallel 35S promoters (R. Kay et al., 1987) and the mas-35S promoters (L. Comai et al., 1990), can also be used. Furthermore, the cauliflower mosaic virus 19S promoter (J. Paszkowski et al., 1984) and scrophularia mosaic virus-derived 34S promoter (M. Sanger et al. , 1990) can also be included. As further examples, there may be mentioned the actin promoter, ribulose-1,5-bisphosphate carboxylase small subunit (rbcS) promoter and so forth, which are plant-derived promoters.

The terminator includes DNA sequences capable of efficiently terminating the transcription of a desired structural gene in plants and includes all terminators functioning in plants that are used in the relevant field of art for the transformation of plants. Specifically, the nopaline synthase (nos) terminator, for example, can be mentioned as a typical example.

The transcriptional activation element of the present invention or the transgene expression cassette comprising said element can be used for inducing or regulating the expression in a plant of the gene introduced, and this widely applies to plants in general.

The plant is not particularly restricted but includes, among others, agriculturally useful plants, whether monocotyledonous or dicotyledonous. Among the monocotyledonous plants, for instance, there are corn, rice, wheat, barley, African or Indian millet, oat, rye, millet and other cereal crop plants as well as lily, orchid, iris, palm, tulip, sedge and other various ornamental plants. The dicotyledons include chrysanthemum, snapdragon, carnation, magnolia, poppy, cabbage, rose, pea, poinsettia, cotton, cactus, carrot, cowberry, peppermint, sunflower, tomato, elm, oak, maple tree, poplar, soybean, melon, beet, rape, potato, lettuce, carica papaya, etc.

The present invention further provides a transgenic plant which contains the transcriptional activation element of the present invention or the transgene expression cassette of the present invention comprising said element and in which the phenomenon of the position effect-due gene silencing (inactivation) has been suppressed for the desired foreign gene Introduced or the transcription of the foreign gene has been activated. Said transgenic plant includes the offspring thereof.

The term "plant" as used herein is intended to include not only a perfect plant body but also a portion of a plant body, such as a leaf, seed, bulb or cutting. It further includes protoplasts, plant calli, mericlones and like plant cells as well.

The method of producing such transgenic plant is not particularly restricted but may be any of those DNA introduction methods which are conventionally used in the relevant field of art. Specifically, it is a method of introducing a DNA into plant cells using an expression plasmid containing the transcriptional activation element of the present invention or a transgene expression cassette containing said element and includes, among others, such known methods as the Agrobacterium method, electric method (eleotroporation) and particle gun method.

The thus-obtained plant cells containing the transcriptional activation element of the invention, the transgene expression cassette containing said element, or the expression plasmid can be regenerated by one of the conventional methods used in the plant tissue culture technology as described, for example, in S. B. Gelvin, R. A. Schilperoot and D. P. S. Verma: Plant Molecular Biology Manual (1991), Kluwer Academic Publishers or Valvekens et al., Proc. Natl. Acad. Sci., DA: 5536-5540 (1988), whereby plant bodies, or portions thereof, derived from said plant cells can be obtained.

The expression plasmid of the present invention may be any one provided that it contains the desired DNA sequence to be introduced (transgene) together with the transcriptional activation element and functional DNA sequences such as a promoter and a terminator. It is preferred, however, that these DNA sequences be operatively joined to one another. The phrase "operatively joined" means that the plasmid functions for the intended purpose. Specifically, it is implied that when the plasmid in question is introduced into plant cells, the desired transgene (structural gene) is expressed under the control of the transcriptional activation element and the expression is efficiently terminated by the action of the terminator, without inactivation of the promoter contained in said plasmid.

The present invention further includes a method of causing expression of a transgene in a plant. Such method can be carried out at least by the step of introducing, into a plant, the transgene expression cassette with the transgene integrated therein, such as mentioned above, and the step of causing expression of said transgene in said plant. The introduction of the transgene expression cassette (DNA) into the plant and the expression of said transgene can both be effected by the techniques per se known in the art (Plant Molecular Biology Manual, 1991, Kluwer Academic Publishers).

### EXAMPLES

The following examples illustrate the present invention in further detail. They are, however, by no means limitative of the scope of the present invention. The genetic engineering techniques and the experimental procedures of molecular biology (restriction enzyme treatment conditions, ligation reaction conditions, transformation into *Escherichia coli,* etc.), which are to be employed in the practice of the present invention, can be carried out as generally and widely employed, for example as described in J. Sambrook, E. F. Frisch, T. Maniatis: Molecular Cloning, 2nd edition, Cold Spring Harbor Laboratory Press, 1989 and D. M. Glover: DNA Cloning, IRL Press, 1985, among others.

### Example 1

### 1) Target plant, target gene

In searching for MITE-like elements, carrot (*Daucus carota* L. cv. Kurodagosun) was used as the target plant, and the phenylalanine ammonia-lyase (PAL) of said carrot as the target gene.

2) Cloning of the carrot PAL genes gDCPAL3 and gDCPAL4 Carrot genomic sequences were cloned from a carrot genomic DNA library. The carrot genomic DNA library was constructed, as previously described by the present inventors (Ozeki, Y., Davies, E. and Takeda, J.: Structure and expression of chalcone synthase gene in carrot suspension cultured cells regulated by 2,4-D. Plant Cell Physiol., 34:

1029-1037 (1993)), from cultured carrot cells (Ozaki, Y. and Komamine, A.: Induction of anthocyanin synthesis in relation to embryogenesis in a carrot suspension culture; Correlation of metabolic differentiation with morphological differentiation. Physiol. Plantarum, 53: 570-577 (1981)) using the *λ* EMBL3 vector (product of Toyobo).

Specifically, carrot genomic DNA was prepared from carrot freeze-dried using a cetyltrimethylammonium bromide (CTAB) solution according to the method of Murray and Thompson (1980) (Murray, M. G. and Thompson, W. F.; Rapid isolation of high molecular weight plant DNA. Nucl. Acids Res. 8: 4321-4325 (1980)). The genomic DNA obtained was partially digested with *Sau*3AI and the digested DNA was fractionated by size by the sucrose density gradient method. The DNA fraction within 15 to 20 kbp was collected and ligated to the *Bam*HI-digested *λ* EMBL3 vector, followed by packaging in phage particles, to thereby construct a carrot nuclear library.

Then, the carrot genomic library was screened (Sambrook et al., 1989) for carrot PAL genomic clones using the PAL cDNA (ANT-PAL cDNA) as the probe cloned by the method of Ozeki et al. (Ozeki, Y., Matsui, K., Sakuta, M., Matsuoka, M., Ohashi, Y., Kano-Murakami, Y., Yamamoto, N, and Tanaka, Y.: Differential regulation of phenylalanine ammonia-lyase genes during anthocyanin synthesis and by transfer effect in carrot cell suspension cultures. Physiol. Plantarum, 80: 379-387 (1990)). The hybridization for screening of the carrot genomic library was effected by overnight treatment at 68°C with a solution containing 6 x SSC, 60 mM sodium phosphate (pH 6.8), 10 mM EDTA, 1% SDS, 0.02% polyvinylpyrrolidone, 0.02% Ficoll 400 and 100 *µ* g/ml denatured salmon sperm DNA. The membrane washing was carried out twice (15 minutes x 2) in a 2 x SSC solution containing 0.5% SDS at room temperature, twice (10 minutes x 2) in a 0.1 x SSC solution or a 1 x SSC solution containing 0.1% SDS at room temperature and, finally, twice (30 minutes x 2) in a 0.1 x SSC solution at 68°C.

As a result, eight positive clones were obtained. Restriction enzyme maps were prepared for such clones and, according to the maps, the clones were classifiable into two genes, which were named *gDCPAL3* and *gDCPAL4,* respectively.

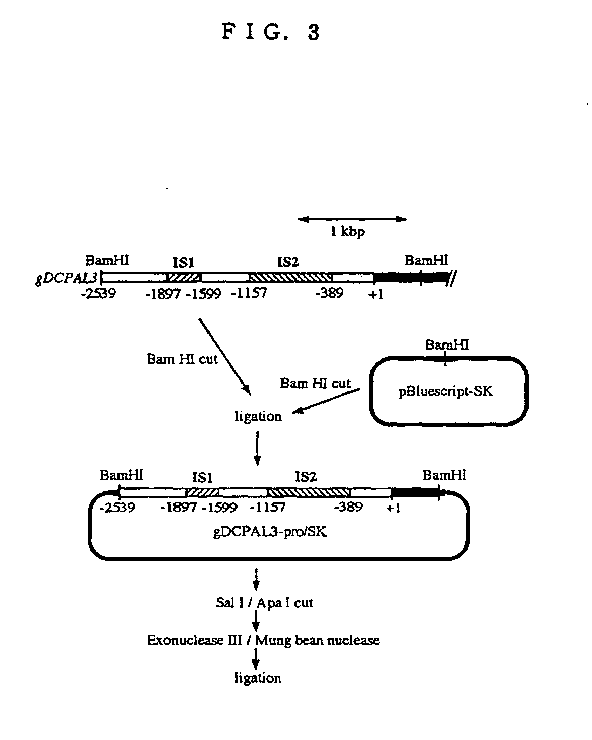

With *gDCPAL3,* the *λ* phage of a positive clone was cultured, *λ* DNA was extracted, cleaved with *Bam*HI and subjected to Southern transfer to a nylon membrane, according to the methods described in Sambrook et al. (1989). With this, Southern analysis was carried out using a probe prepared from the above-mentioned ANT-PAL cDNA by cleavage with *Eco*RI and labeling a DNA fragment (984 bp) thereof at the 5' end with [³²p], and a 2.77 kbp DNA fragment hybridizing with said probe was obtained. This DNA fragment was cleaved with *Bam*HI and subcloned in the pBluescript SK plasmid treated with calf intestine alkaline phosphatase (CIP), to give gDCPAL3-pro/SK (cf. Fig. 3). Then, the plasmid gDCPAL3-pro/SK obtained was cleaved with the restriction enzymes *Sal*I and *Apa*I, a series of deletion DNA fragment groups were produced using exonuclease III and mung bean nuclease by the method described by Sambrook et al. (1989), and the nucleotide sequence of the *gDCPAL3* DNA was determined using them. The site of transcription initiation (+1) was determined based on the positions of bands as found by the primer extension method using mRNA extracted from carrot as described by Ozeki and Takeda (1994) (Regulation of phenylalanine ammonia-lyase genes in carrot suspension cultured cells. Plant Cell. Tissue and Organ Culture, **38**: 221-225 (1994)).

With *gDCPAL4,* the plasmid gDCPAL4-pro/SK was produced and the nucleotide sequence of the *gDCPAL4* DNA corresponding to the above was determined in the same manner. Specifically, the *λ* DNA obtained from the *λ* phage of a positive *gDCPAL4* clone was cleaved with *Hind*III and *Bam*HI, Southern analysis was performed using the same probe as mentioned above, and a 1.63 kbp DNA fragment hybridizing with the probe was cleaved with *Hind*III and *Bam*HI and subcloned in the pBluescript SK plasmid treated with CIP, to give gDCPAL4-pro/SK. Then, the thus-obtained plasmid gDCPAL4-pro/SK was cleaved with the restriction enzymes *Xba*I and *Bst*XI, a series of deletion DNA fragment groups were produced using exonuclease III and mung bean nuclease by the method described by Sambrook et al. (1989), and the nucleotide sequence of the *gDCPAL4* DNA was determined using them.

### 3) Results

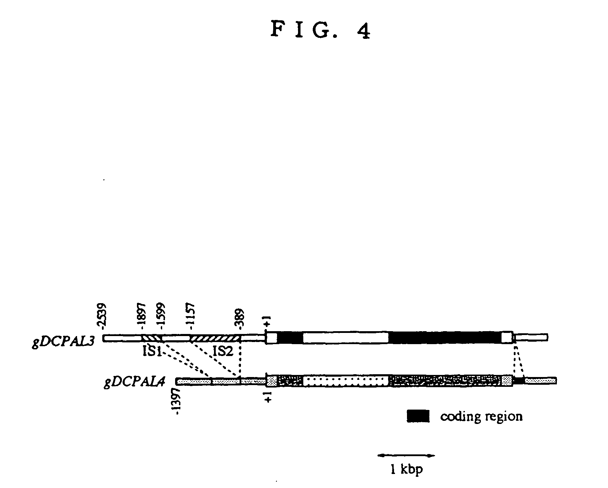

Comparison of the nucleotide sequences of *gDCPAL3* and *gDCPAL4* revealed that the promoter region of *gDCPAL3* has miniature inverted-repeat transposable elements (MITEs) having imperfect inverted repeat sequences not found in *gDCPAL4* were present at two sites, namely -1897 to -1599 (299 bp in length) and -1157 to -389 (769 bp in length) (Fig. 4). These sequences were named IS1 and IS2, respectively.

These sequences and the nucleotide sequences around the sites of insertion were sequenced using an autosequencer (product of LICOR model 4000L). The nucleotide sequence of IS1 is shown under SEQ ID N0:2 and the nucleotide sequence of IS1 under SEQ ID NO: 1.

The characteristics of these IS1 and IS2 were as follows:

### (1) IS1

It had the nucleotide sequence shown under SEQ ID NO:2 (total length: 299 bp), had inverted repeat sequences (32 bp), which were imperfect to each other, in the 5' and 3' terminal regions, and had a target duplication sequence, TA, at the site of insertion into the genome serving as the target gene. Based on these facts, it was estimated to be the gene sequence of a novel MITE element belonging to the family *Stowaway* already reported (Bureau, T. E. and Wessler, S. R. *Stowaway:* a new family of inverted repeat elements associated with the genes of both monocotyledonous and dicotyledonous plants. Plant Cell, **6**: 907-16 (1994)). The stem structure of the element IS1 and the structure of the terminal inverted repeat sequence region and of the insertion site region are shown in Fig. 2.

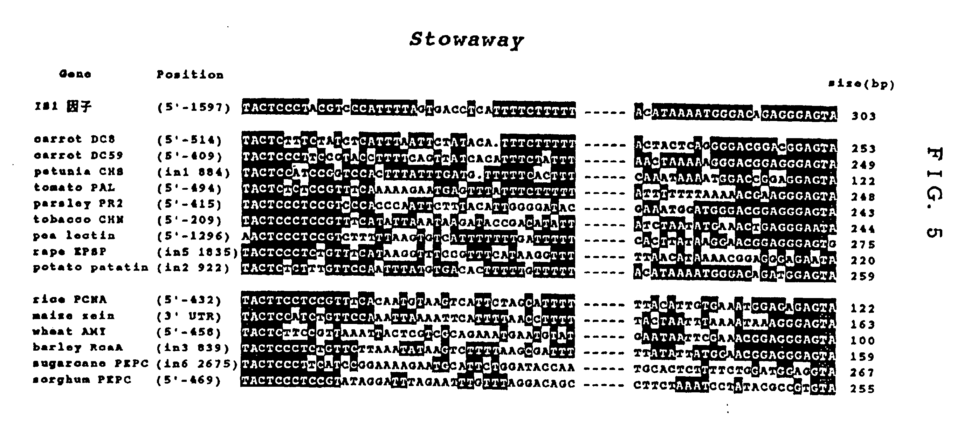

Based on the nucleotide sequence information obtained, homology analysis of the nucleotide sequence was performed using commercial databases (e.g. GENE TYX-MAC/CD1995), whereupon, in the terminal inverted repeat sequences, 70-90% homology was found with the gene sequence of MITE elements belonging to the *Stowaway* family (Bureau and Wessler (1994)). It was thus confirmed that said element is a transposable element belonging to the *Stowaway* family (Fig. 5).

### (2) IS2

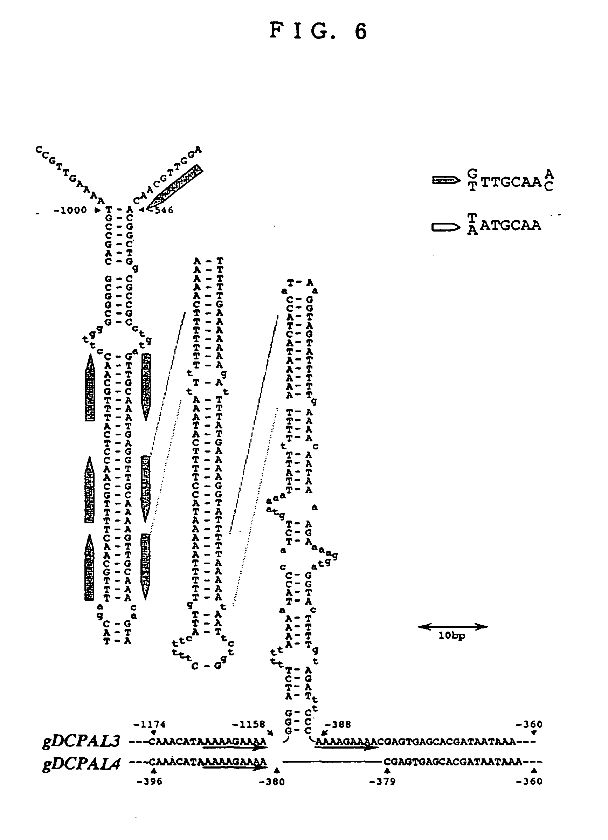

It had the nucleotide sequence shown under SEQ ID NO:1 (total length: 769 bp), had inverted repeat sequences (158 bp), which were imperfect to each other, in the 5' and 3' terminal regions, and had a target duplication sequence, AAAAGAAAA, at the site of insertion into the genome serving as the target gene. Homology comparison of the nucleotide sequence was made but no homology with known transposable elements was detected. It was thus found that it is a transposable element, particularly a MITE-like element, constituting a novel family belonging to none of the so far known transposable element families. The overall structure (stem structure) of the IS2 element is shown in Fig. 1 and the structure (nucleotide sequence) of its terminal inverted repeat sequence region and of the insertion site region in Fig. 6.

### Example 2

### (1) Cloning of IS1, IS2, IS12 and MU3

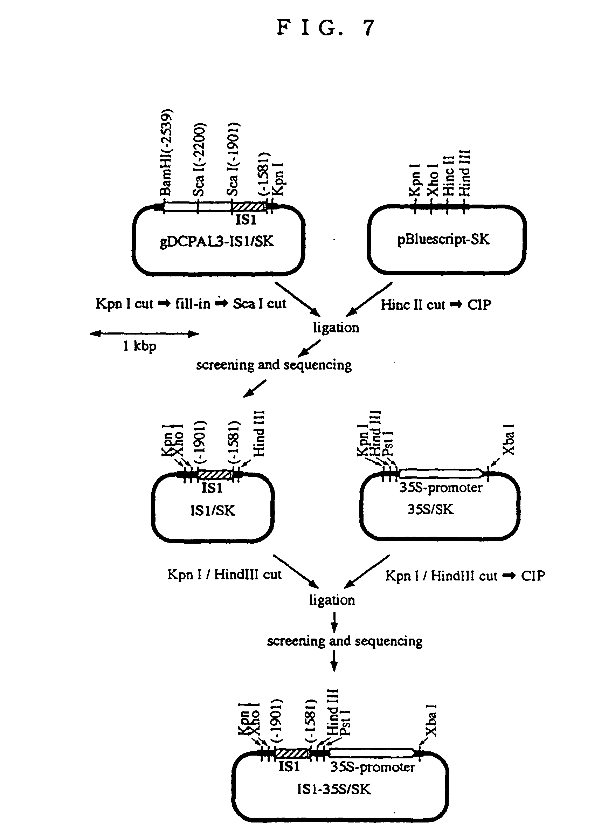

### (i) Cloning of IS1 (Fig. 7)

From among the plasmids having a deletion DNA fragment derived from the 3' terminus of the *gDCPAL*3 promoter region as prepared for nucleotide sequence determination in Example 1, a plasmid (gDCPAL3-IS1/SK) with deletion to -1581 was selected, and a 321 bp DNA fragment was excised by cleaving that plasmid with *Kpn*I, rendering blunt-ended using T4 DNA polymerase and cleaving with *Sca*I*,* followed by agarose gel electrophoresis. This was subcloned in the plasmid pBluesoript SK cleaved with *Hinc*II and treated with CIP. Plasmids were extracted from among *Escherichia coli* colonies harboring a plurality of independent clones obtained by the above subcloning and the nucleotide sequences thereof were determined for revealing the direction of each DNA fragment inserted. In this way, a plasmid with the 5' terminal side of IS1 being inserted on the *Kpn*I side of the multiple cloning site of the pBluesoript SK plasmid was selected and named IS1/SK. Further, the cauliflower mosaic virus 35S promoter (35S) fragment obtained by cleavage of pBI221 (Clontech Inc.) with *Hind*III and *Sma*I was recovered by agarose gel electrophoresis and subcloned in the pBluescript SK plasmid cleaved with *Hind*III and *Sma*I and treated with CIP, to give a plasmid named 35S/SK. From IS1/SK, the insert DNA fragment was excised by cleaving with *Kpn*I and *Hind*III, followed by agarose gel electrophoresis, and this was subcloned in the 35S/SK plasmid cleaved with *Kpn*I and *Hind*III and treated with CIP, to give IS1-35S/SK having the IS1 region in the upstream region of the 35S promoter region.

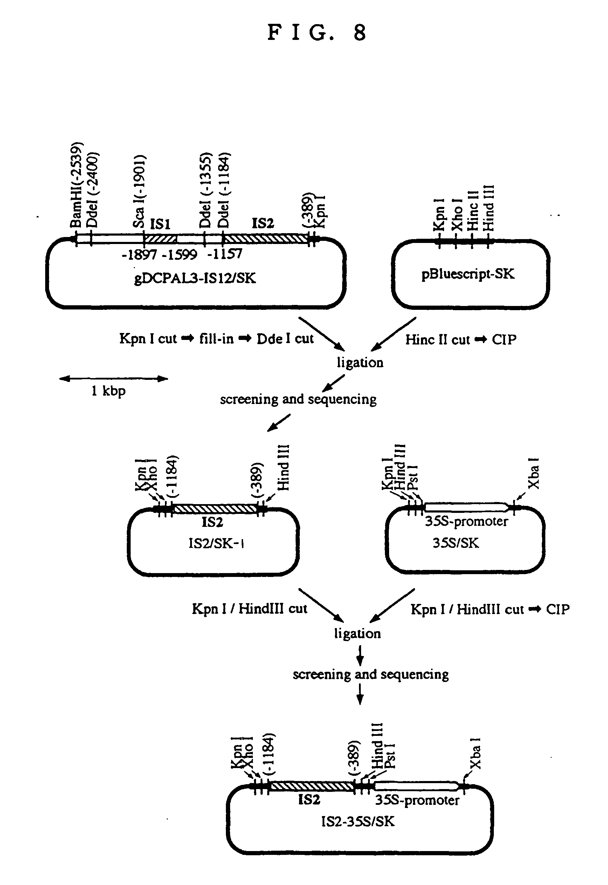

### (ii) Cloning of IS2 (Fig. 8)

From among the plasmids having a deletion DNA fragment derived from the 3' terminus of the *gDCPAL3* promoter region as prepared for nucleotide sequence determination in Example 1, a plasmid (gDCPAL3-IS12/SK) showing deletion to -389 was selected, and a 797 bp DNA fragment was excised by cleaving that plasmid with *Kpn*I, rendering blunt-ended using T4 DNA polymerase and cleaving with *Dde*I, followed by agarose gel electrophoresis. This was subcloned in the plasmid pBluescript SK cleaved with *Hinc*II and treated with CIP. Plasmids were extracted from among *E*. *coli* colonies harboring a plurality of independent clones obtained by the above subcloning and the nucleotide sequences thereof were determined for revealing the direction of each DNA fragment inserted. In this way, a plasmid with the 5' terminal side of IS2 being inserted on the *Kpn*I side of the multiple cloning site of the pBluescript SK plasmid was selected and named IS2/SK-1. One with the 3' terminal side of IS2 being inserted on the *Kpn*I side, namely in the reverse direction, was selected and named IS2/SK-2 (reverse).

IS2/SK-1 was cleaved with *Kpn*I and *Hind*III and the insert DNA fragment was recovered by agarose gel electrophoresis, and this was subcloned in the 35S/SK plasmid cleaved with *Kpn*I and *Hind*III and treated with CIP, to give IS2-35S/SK having the IS2 region (positive strand) in the upstream region of the 35S promoter region.

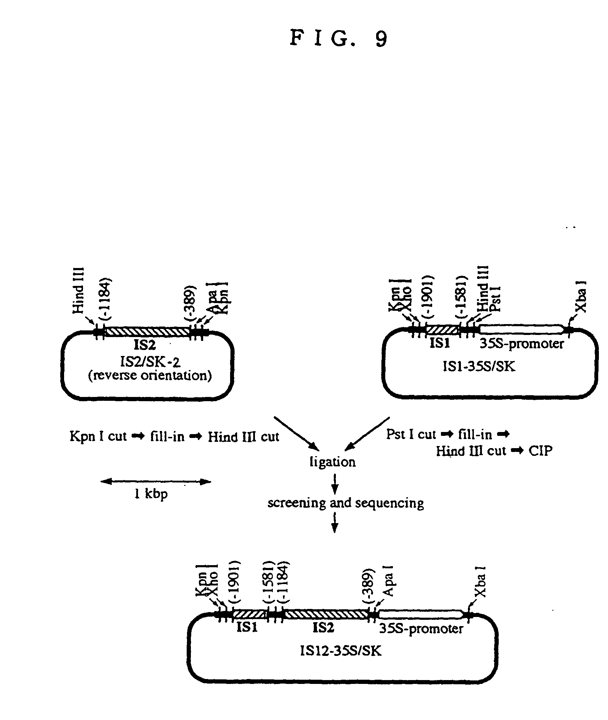

### (iii) Cloning of IS12 (IS1-IS2 tandem coupling product) (Fig. 9)

The IS2/SK-2 (reverse) obtained as described above under (ii) was cleaved with *Kpn*I, rendered blunt-ended using T4 DNA polymerase and then cleaved with *Hind*III, and the insert DNA fragment was recovered by agarose gel electrophoresis. This was cleaved with *Pst*I, rendered blunt-ended using T4 DNA polymerase and then subcloned in the IS1-35S/SK plasmid cleaved with *Hind*III and treated with CIP, to give IS12-35S/SK having the IS1 region and IS2 region in the upstream region of the 35S promoter region in a tandem manner.

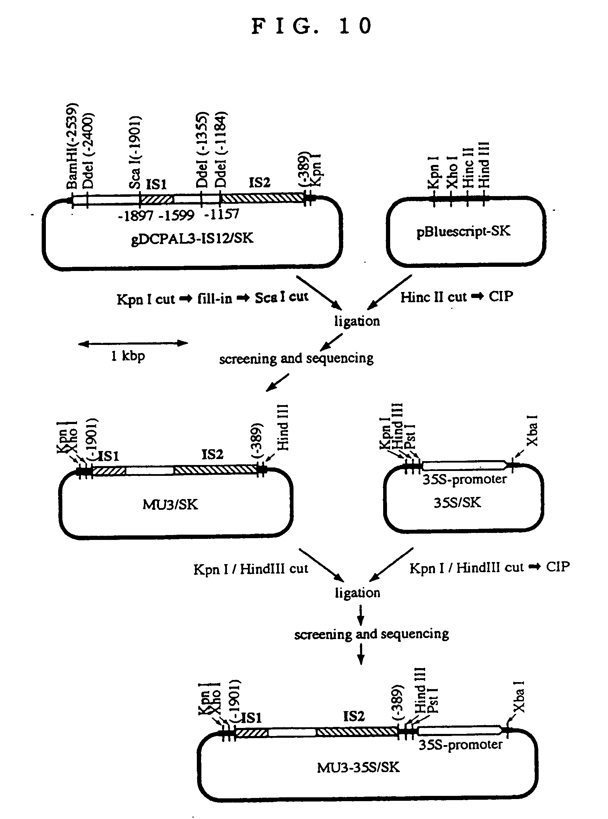

### (iv) Cloning of MU3 (Fig. 10)

As for MU3, gDCPAL3-IS12/SK was cleaved with *Kpn*I, then rendered blunt-ended using T4 DNA polymerase and cleaved with *Sca*I, and a 1,514 bp DNA fragment was recovered by agarose gel electrophoresis. This was subcloned in the pBluescript SK plasmid cleaved with *Hinc*II and treated with CIP. Plasmids were extracted from a plurality of *E. coli* colonies harboring each independent clone as obtained by the above subcloning and the nucleotide sequences thereof were determined to thereby check the direction of DNA fragment insertion. A plasmid with the 5' terminal side of IS1 inserted on the *Kpn*I side of the multicloning site of the pBluescript SK plasmid was selected and named MU3/SK. The insert DNA fragment was excised by cleaving MU3/SK with *Kpn*I and *Hind*III, followed by agarose gel electrophoresis. This was subcloned in the 35S/SK plasmid cleaved with *Kpn*I and *Hind*III and treated with CIP, to give MU3-35S/SK having the IS1 region and IS2 region, via a *gDCPAL3-*derived region sequence (441 bp), in the upstream region of the 35S promoter region.

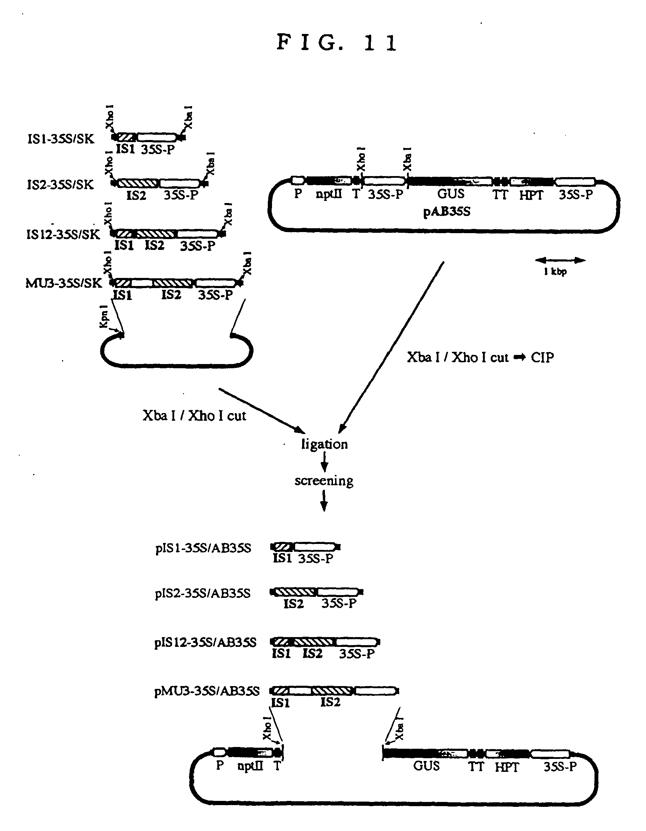

### (2) Insertion of IS1, IS2, IS12 and MU3 into a plant gene expression vector (Fig. 11)

pABN-Hm1 (Mita, S., Suzuki-Fujii, K. and Nakamura, K. Sugar-induoible expression of a gene for β-amylase in Arabidopsis thaliana. Plant Physiol., 107: 895-904 (1995); gift from Dr. Kenzo Nakamura at Nagoya Univeristy) was cleaved with *Hind*III to thereby excise the *β* -amylase promoter (1.7 kb), which was rendered blunt-ended using T4 DNA polymerase, then cleaved with *Xba*I, treated with CIP and then subjected to agarose gel electrophoresis, whereby a 10 kbp DNA fragment containing the Ti plasmid region as well as the kanamycin resistance gene [nos promoter/coding region of neomycin phosphotransferase II gene (nptII)/nos terminator], the coding region (GUS)/nos terminator of the *β-*glucuronidase gene, and the hygromycin resistance gene [35S promoter/coding region (HPT) of hygromycin phosphotransferase gene/nos terminator] was isolated. In this was subcloned a 35S fragment obtained from 35S/SK by cleavage with *Hind*III, rendering blunt-ended using T4 DNA polymerase and cleaving with *Xb*aI, to construct pAB35S.

This was cleaved with *Xho*I and *Xba*I and treated with CIP and then a vector was prepared by cutting off the 35S DNA fragment by agarose gel electrophoresis. Separately, the IS1-35S/SK, IS2-35S/SK, IS12-35S/SK and MU3-35S/SK prepared as mentioned above were each cleaved with *Xho*I and *Xba*I and DNA fragments for insertion (transgene expression cassettes) were recovered by agarose gel electrophoresis. Such DNA fragments were ligated to the vector mentioned above and used to transform *E*. *coli* DH5α. LB agar medium containing 25 *µ*g/L of kanamycin (1% Bacto-Trypton, 0.5% yeast extract, 1% sodium chloride, 1.5% agar powder for bacterial culture media) was sowed with each of the E. *coli* transformants obtained, and plasmids were extracted from the colonies obtained by the rapid plasmid DNA extraction method and the restriction enzyme maps of the plasmids obtained were checked, whereby the formation of the constructs pIS1-35S/AB35S, pIS2-35S/AB35S, pIS12-35S/AB35S and pMU3-35S/AB35S, namely the constructs with the above transgene expression cassettes respectively inserted between the nptII gene responsible for kanamycin resistance and the GUS gene, which is a structural gene, was confirmed as shown in Fig. 11.

### (3) Production of competent cells of Agrobacterium tumefaciens

A YEP solid medium (prepared by adding powdered agar for bacterial culture media to YEP medium comprising 1% yeast extract, 1% Bactoheptone and 0.5% sodium chloride to a concentration of 1.5%, followed by solidification by autoolaving; hereinafter the same shall apply) was smeared with a loopful of cells taken from a glycerol stock of *A*. *tumefaciens* EHA 101 and the cells were cultured at 28° C in the dark for 2 days. Grown single colonies of A. *tumefaciens* were each collected with a toothpick and sowed in 1.5 ml of YEP medium and shake-cultured overnight at 28° C. 80 ml of YEP medium was placed in a 500-ml flask, 0.8 ml of the A. *tumefaciens* culture fluid was added, and shake culture was performed at 28° C until OD₆₀₀ = 0.4. This was cooled with ice, transferred to a centrifuge tube ice-cooled in advance, and centrifuged at 6,000 rpm at 4° C for 5 minutes, the supernatant was removed, and 20 ml of 10% glycerol was added to suspend the sediment. This procedure was repeated three times, and the medium was completely removed to give competent cells of A. *tumefaciens.* For stocking, the cells were suspended in 400 µl of 10% glycerol and the suspension was distributed in 40-*µ*l portions into tubes, followed by rapid freezing in liquefied nitrogen.

### (4) Introduction of plasmid DNAs into A. tumefaciens

The constructs obtained as described above under (2) (plasmids pIS1-35S/AB35S, pIS2-35S/AB35S, pIS12-35S/AB35S and pMU3-35S/AB35S) were each introduced into the competent cells of A. *tumefaciens* by electoporation (using Shimadzu GTE-10). Specifically, about 100 ng each of the plasmids prepared as described above under (2), namely pIS1-35S/AB35S (IS1), pIS2-35S/AB35S (IS2), pIS12-35S/AB35S (IS12) and pMU3-35S/AB35S (MU3), and the plasmid pAB35S (35S) to serve as a control with no insertion of the tanscriptional activation element(s) (IS1 and/or IS2) was admixed with 40 *µ*l of competent cells prepared as described above under (3), and each mixture was transferred to an eleotroporation cell. Electric pulses (1.2 kV, 35 *µ* F, 550 Ω) were given, and 1 ml of YEP medium was immediately added, and incubation was performed at 28° C for 1 hour. About 50 *µ*l was taken and YEP solid medium containing 50 *µ*g/L of hygromycin was smeared therewith, and incubation was performed in the dark at 28°C for 2 days. A monocolony that had grown was again spread lightly over another portion of YEP solid medium containing 50 *µ*g/L of hygromycin using a platinum loop and incubated at 28° C for 24 hours. A portion of cells were taken and planted in 5 ml of YEP medium containing 50 *µ*g/L of hygromycin and shake-cultured overnight at 28°C.

### Example 3

### (1) Introduction of transcriptional activation element-containing constructs into tobacco cultured cells

Using A. tumefaciens with the construct prepared in Example 2, namely pIS1-35S/AB35S (IS1), pIS2-35S/AB35S (IS2) or pIS12-35S/AB35S (IS12), introduced therein (hereinafter referred to as "transformant *A*. *tumefaciens*"), the constructs IS1, IS2 and IS12 were respectively introduced into tobacco cultured cells. In a control run, *A*. *tumefaciens* with pAB35S (35S) introduced therein was used and the same procedure was followed.

First, the above transformant A. *tumefaciens* cultured in 5 ml of YEP liquid medium was transferred to a 50-ml centrifuge tube and centrifuged at 3,000 rpm for 10 minutes. The supernatant was discarded, 25 ml of Linsmaier & Skoog medium (Linsmaier, E. M. and Skoog, F.; Physiol. Plantarum **18**, 100-127 (1965); hereinafter referred to as "Lins medium") was added to the sediment and, after resuspending, centrifugation was carried out again at 3,000 rpm and at room temperature for 10 minutes, and the supernatant was discarded. This procedure was repeated four times. Cells of A. *tumefaciens* were harvested, Lins medium was added for suspending in an amount to give OD₆₀₀ = 0.2, acetosyringone was added thereto to a concentration of 10 *µ*g/ml, followed by resuspending.

Saparately, cultured tobacco cells BY-2 (gift from Dr. Toshiyuki Nagata at University of Tokyo) to be used for introduction of each construct were cultured beforehand in 45 ml of Lins medium containing 2,4-dichlorophenoxyacetic acid (2,4-D), 1 ml of the cell-containing suspension culture fluid was transferred to a fresh portion of Lins medium at one-week intervals, and cells that had entered the logarithmic growth phase after the lapse of about 100 hours following transfer were used.

A sterile 90-mm dish was sowed with said tobacco cultured cells BY-2 (4 ml), and 100 µl of cells of transformant A. *tumefaciens* washed by the above procedure were added uniformly onto the tobacco cultured cells. After slight blending, co-culture was carried out in the dark at 22°C for 3 days.

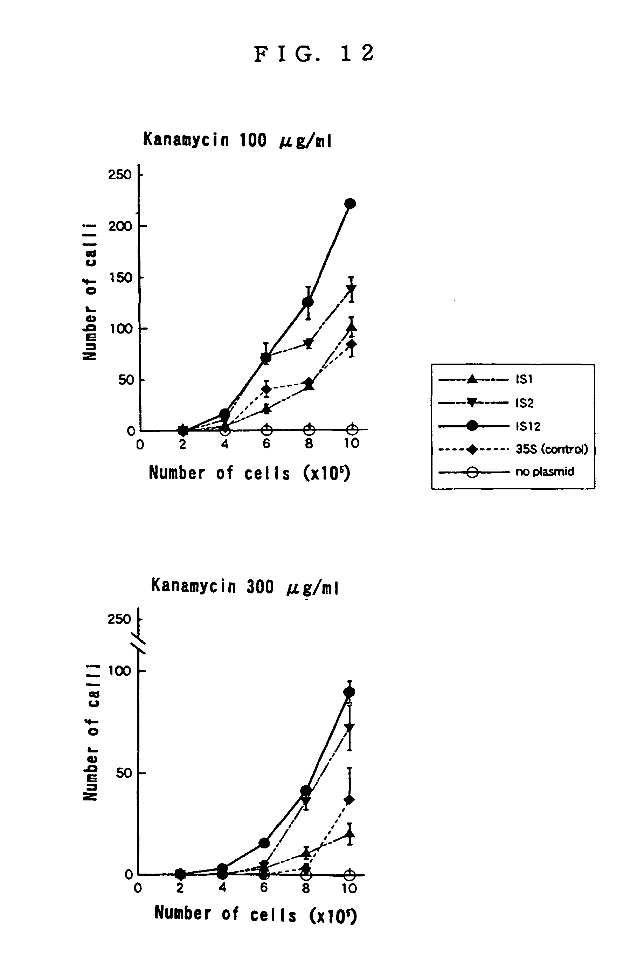

Then, 12 ml of Lins medium was added to the cultured cell fluid for suspending, the suspension was transferred to a 50-ml centrifuge tube and centrifuged at 1,000 rpm for 1 minute, and the supernatant was discarded. This procedure was repeated four times. Then, 12 ml of Lins medium containing 250 *µ*g/ml of claforan was added and the same procedure as mentioned above was once more repeated. After discarding the supernatant, about 25 ml of Lins medium was added to the sediment cells to thereby suspend them, and the cells in 0.25 µl of the medium were counted using a hemocytometer. The cells were uniformly sowed onto portions of Lins solid selection medium containing 100 *µ*g/ml or 300 *µ* g/ml of kanamycin (further containing 250 *µ*g/ml of claforan) so that the number of cells per plate amounted to 4 x 10⁵, 6 x 10⁵, 8 x 10⁵, 10 x 10⁵ or 15 x 10⁵. They were cultured in the dark at 28° C. After one month of culture, the number of transgenic cultured tobacco cells (transformant calli) formed on each plate and the formation rate or yield were determined. The results thus obtained are shown in Table 1 and Fig. 12.

**Table 1**

| Number of transformant calli formed from cultured tobacco cells BY-2 in kanamycin-containing medium and rate of transformant callus formation | | | | |
|---|---|---|---|---|
| Kanamycin 100 *µ*g/ml | | | | |
| Number of cells (x 10⁵) | 4 | 6 | 8 | 10 |
| IS1 | 4±0 | 20±4 | 43±3 | 99±10 |
| | (0.10) | (0.33) | (0.54) | (0.99) |
| IS2 | 10±7 | 72±13 | 84±4 | 137±12 |
| | (0.25) | (1.20) | (1.05) | (1.37) |
| IS12 | 16±3 | 70±6 | 124±16 | 220±4 |
| | (0.40) | (1.67) | (1.55) | (2.20) |
| 35S | 3±2 | 40±8 | 47±2 | 83±13 |
| (Control) | (0.08) | (0.67) | (0.59) | (0.83) |

| Kanamycin 300 *µ*g/ml | | | | |
|---|---|---|---|---|
| Number of cells (x 10⁵) | 4 | 6 | 8 | 10 |
| IS1 | 0 | 3±0 | 10±3 | 20±5 |
| | (0.00) | (0.05) | (0.13) | (0.20) |
| IS2 | 0 | 4±2 | 36±4 | 72±11 |
| | (0.00) | (0.07) | (0.45) | (0.72) |
| IS12 | 2±1 | 15±1 | 41±2 | 89±5 |
| | (0.05) | (0.25) | (0.51) | (0.89) |
| 35S | 0 | 0 | 3±2 | 37±16 |
| (Control) | (0.00) | (0.00) | (0.04) | (0.37) |

| | | | | |
|---|---|---|---|---|
| Upper: Number of calli formed | | | | |
| Lower: Yield (x 10⁻² %) | | | | |

From the above results, it was found that insertion of the construct IS2 or IS12 into tobacco cultured cells increases the yield of transformant calli in kanamycin-containing medium and that the yield is higher than the yield of transformant calluses from cultured tobacco cells (35S) without insertion of such element. Specifically, the yield of transformant calli (transgenic cultured tobacco cells) with the construct IS2 or IS12 introduced therein was 1.6 to 2.6 times higher as compared with the control (35S) without introduction of such element.

Particularly when the kanamycin concentration in medium was 300 *µ*g/ml, it was observed that, by introducing the construct IS2 or IS12, the yield of transformant calli is increased to a level 10 times or more higher as compared with the control (35S). This suggests that insertion of the construct IS2 or IS12 result in an increased level of expression of the ntpII gene responsible for kanamycin resistance and occurring in the vicinity or marginal region of said construct.

### (2) GUS activity measurement in transformant tobacco calli

Based on the above results, the expression of the GUS gene, which is a reporter, was checked using the above pIS12-35S/AB35S (IS12) as the construct, to thereby check whether the above inserted construct can increase the expression of the structural gene.

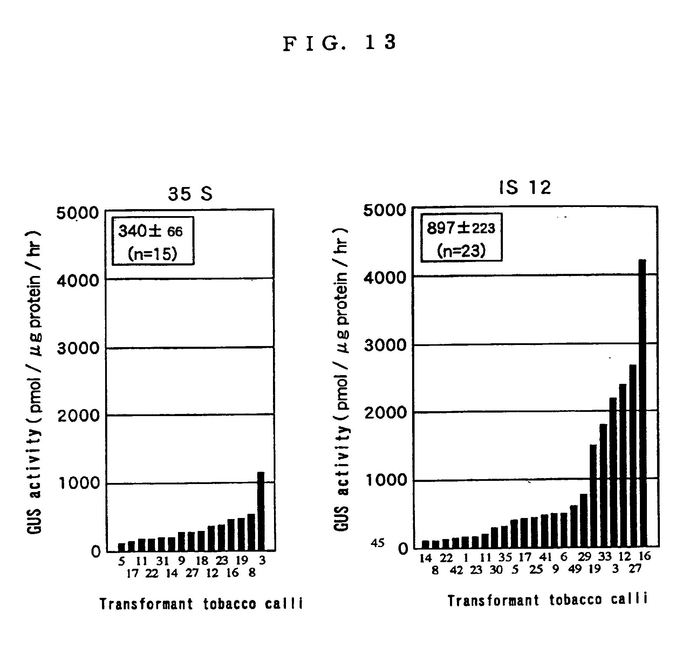

The expression of the GUS gene was examined by first randomly selecting independent calli from among a plurality of transformant tobacco calli, extracting nuclear DNA from each callus and, after confirming the gene introduction by Southern analysis, extracting proteins from the callus and measuring the GUS activity. Specifically, 0.75 g of the transformed tobacco callus was taken and proteins were extracted using GUS-Light (Tropix, Inc.). The protein concentration was determined using Bio-Rad's protein assay kit and then, using GUS-Light (Tropix) and the luminometer Lumat LB905 (Bertold Japan), the GUS activity was measured for 5 seconds. The results thus obtained are shown in Fig. 13. In the figure, the GUS activity (ordinate) is shown in terms of luminescence value per unit weight of protein as calculated by dividing the luminescence value obtained from the luminometer by the protein weight.

As is evident from Fig. 13, the transformant tobacco calli resulting from insertion of the construct IS12 showed, on an average, about 2.6 times higher GUS activity as compared with the control resulting from insertion of element-free pAB35S (35S) . From this, it was found that the expression of the GUS gene is significantly increased by insertion of the construct IS12.

This indicates that each element (IS1 element, IS2 element, and a coupling product therefrom (e.g. IS12 element)) contained in the above constructs is a transcriptional activation element.

### Example 4 Introduction of a transcriptional activation element-containing construct into a tobacco plant (leaf disk method)

Using A. *tumefaciens* with the construct prepared in Example 2, namely pIS1-35S/AB35S (IS1), pIS2-35S/AB35S (IS2) or pIS12-35S/AB35S (IS12), introduced therein (hereinafter referred to as "transformant *A*. *tumefaciens*"), the constructs IS1, IS2 and IS12 were respectively introduced into tobacco leaves by the leaf disk method. In a control run, *A*. *tumefaciens* with the construct-free pAB35S (35S) introduced therein was used and the same procedure was followed.

Specifically, each tobacco (SR 1) leaf was immersed in a 10% hypochlorous acid solution, air bubbles were removed using a medicine spoon over 2 minutes with gentle stirring by means of a stirrer, the solution was renewed and the same procedure was repeated for further 5 minutes. The leaf was taken out and immersed in sterilized water, followed by gentle stirring. While replacing the hypochlorous acid solution with a fresh portion, the same procedure was repeated three times in all. After removing the moisture using a sterilized paper towel, the leaf was punched with a cork borer to give a leaf disk (the vein being removed). This was immersed in 10 ml of sterilized water. The thus-prepared leaf disk was immersed in the above-mentioned transformant *A*. *tumefaciens* cultured in 5 ml of YEP medium (adjusted to OD₆₀₀ = 0.25 with sterilized water). Then, the bacterial suspension and the leaf disk were together emptied onto a sterilized paper towel and the moisture was removed with another sterilized paper towel.

The leaf was placed, inside out, on MS infection medium prepared by supplementing MS medium (Murashige, T. and Skoog, F.; Physiol. Plantarum 15, 473-497 (1962)) with 40 mg/L acetosyringone and 0.2% gelan gum, and cultured in the dark at 25° C for 2 days. Then, each leaf disk was deprived of bacterial cells in the manner of wiping with MS differentiation medium (MS medium supplemented with 0.1 mg/L α-naphthaleneacetic acid, 1 mg/L benzyladenine, 150 mg/L kanamycin, 500 mg/L claforan and 0.2% gelan gum), then placed, inside out, on another portion of MS differentiation medium and cultured at 25°C. The medium was replaced with a fresh portion of MS differentiation medium at two-week intervals and, after the lapse of one month, regenerated shoots were counted. The results thus obtained are shown in Table 2.

**Table 2**

| Comparison in number of regenerated shoots on tobacco leaf disks | | | | |
|---|---|---|---|---|
| Number of shoots per disk | 35S | IS1 | IS2 | IS12 |
| 0 | 73 | 49 | 56 | 50 |
| 1 | 26 | 21 | 20 | 23 |
| 2 | 13 | 17 | 11 | 14 |
| 3 | 10 | 14 | 8 | 5 |
| 4 | 3 | 6 | 9 | 8 |
| 5 | 2 | 1 | 2 | 1 |
| 6 | 1 | 3 | 3 | 2 |
| 7 | 0 | 1 | 2 | 5 |
| 8 | 1 | 0 | 0 | 3 |
| 9 | 0 | 0 | 0 | 1 |
| 10 | 0 | 0 | 2 | 1 |
| 11 | 0 | 0 | 0 | 1 |
| 12 | 0 | 0 | 0 | 1 |
| 13 | 0 | 0 | 0 | 0 |
| 14 | 0 | 0 | 0 | 2 |
| Total number of disks | 129 | 112 | 113 | 117 |
| Total number of shoots | 118 | 151 | 164 | 244 |
| Average number of shoots per disk | 0.91 | 1.35 | 1.45 | 2.09 |

As is evident from the above results, the shoot regeneration efficiency was about 1.4 times when the tobacco plant contained the construct IS1 or IS2 as compared with the element-free control (35S) and, in particular when it contained both IS1 and IS2 in a tandem manner (IS12), about twice as many shoots were obtained as compared with the control. From this, it is evident that the following elements (IS1 element, IS2 element and coupling products obtained therefrom (IS12 and the like)) can increase the activity of the kanamycin gene (ntpII gene) occurring in the vicinity or marginal region of said elements in the tobacco plant. This result supports the judgment drawn in Example 3 that the elements of the present invention are transcriptional activation elements.

### Example 5 Introduction of transcriptional activation element-containing constructs into carrot somatic embryos

Using A. *tumefaciens* with the construct prepared in Example 2, namely pIS1-35S/AB35S (IS1), pIS2-35S/AB35S (IS2), pIS12-35S/AB35S (IS12) or pMU3-35S/AB35S (MU3), introduced therein (hereinafter referred to as "transformant *A*. *tumefaciens"),* the constructs IS1, IS2, IS12 and MU3 were respectively introduced into carrot somatic embryos. In a control run, A. *tumefaciens* with the construct-free pAB35S (35S) introduced therein was used and the same procedure was followed.

Specifically, first, carrot hypocotyls germinated under the sterilized condition were out to a length of about 1 cm, then placed in MS medium containing 4.5 x 10⁻⁶ M 2,4-D (2,4-dichlorophenoxyacetic acid) and cultured in the dark for 24 hours, then placed in 2,4-D-free MS medium and cultured in the dark for 3 days. The medium was replaced with a fresh portion and cultivation was performed in the same manner for 7 days to initiate carrot somatic embryos on the hypocotyls.

Separately, a culture of the above transformant ***A. tumefaciens*** cultured in 5 ml of YEP medium was centrifuged at 3,000 rpm for 10 minutes, the supernatant was removed, and about 30 ml of MS medium was added to suspend the cells. This procedure was repeated twice and the YEP medium was completely removed. Then, centrifugation was carried out at 3,000 rpm for 10 minutes, the supernatant was removed, and MS medium containing 10 mg/L of aoetosyringone was added to the sediment to thereby adjust to OD₆₀₀ = about 0.3.

To this were added the above carrot hypocotyls collected using a net, and the mixture was shaken gently for 5 minutes. The hypocotyls were deprived of the moisture by wiping with a sterilized paper towel and immersed in MS medium containing 10 mg/L of aoetosyringone and cultured in the dark at 22°C for 3 days. The hypocotyls were deprived of the moisture by wiping with a sterilized paper towel and immersed in MS medium containing 500 *µ* g/L of carbenicillin and washed with the medium by shaking gently. After removing the moisture in the same manner, the hypocotyls were cultured in MS agar medium (containing 0.8% agar) containing 500 *µ*g/L of carbenicillin and 100 *µ*g/L of kanamycin in the dark. After 1.5 to 3 months, hypocotyls that had each regenerated a callus were counted. The results thus obtained are shown in Table 3.

**Table 3**

| Comparison in number of callus-regenerating hypocotyls | | | | | | |
|---|---|---|---|---|---|---|
| | | 35S | IS1 | IS2 | IS12 | MU3 |
| + 2,4-D | Number of callus-regenerating hypocotyls | 5 | 6 | 10 | 9 | 8 |
| | Total number of hypocotyls | 78 | 56 | 60 | 61 | 63 |
| | Percent regeneration(%) | 6.4 | 11 | 17 | 15 | 13 |
| - 2,4-D | Number of callus-regenerating hypocotyls | 3 | 9 | 12 | 6 | 4 |
| | Total number of hypocotyls | 77 | 62 | 62 | 64 | 58 |
| | Percent regeneration (%) | 3.9 | 13 | 19 | 9.4 | 6.9 |

As is evident from Table 3, with carrot somatic embryos as well, like the case of tobacco calli, the regeneration efficiency was found improved upon insertion of the construct IS1, IS2, IS12 and MU3 under the dedifferentiational growth conditions of culturing in 2,4-D-containing medium (+ 2,4-D) as well as under the differentiational growth conditions of culturing in 2,4-D-free medium (- 2,4-D). The greatest improvement in regeneration efficiency was observed in the case of the construct IS2 inserted. Example 6 Introduction of transcriptional activation element-containing constructs into rice

Using **A.** *tumefaciens* with the construct prepared in Example 2, namely pIS1-35S/AB35S (IS1) or pIS2-35S/AB35S (IS2), Introduced therein (hereinafter referred to as "transformant A. *tumefaciens"),* the constructs IS1 and IS2 were respectively introduced into rice seeds. In a control run, A. *tumefaciens* with the element-free pAB35S (35S) introduced therein was used and the same procedure was followed.

Specifically, from among fully ripened rice seeds (Nihonbare), those normal in shape and color, among others, were first selected and dehulled by lightly rubbing in a mortar. Dehulled seeds were placed in a 50-ml Falcon tube, 2.5% sodium hypochlorite was added, and the mixture was shaken at 100-120 rpm for 20 minutes. Then, the supernatant was discarded, sterilized water was added, and the mixture was shaken gently. After three repetitions of this procedure, the seeds were placed on callus induction medium and cultured in the dark at 28°C.

After 3 to 4 weeks, among calli formed from scutella and having growing yellowed shoots, only those having a diameter of 2-3 mm and looking like scattered in a group of several were placed on fresh callus induction medium and cultured in the dark at 28°C for 7 days.

Separately, the above transformant A. *tumefaciens* was planted in YEP solid medium and cultured in the dark at 28° C for 3 days. Cells of A. *tumefaciens* were scratched off with a medicine spoon and added to AAI medium (Toriyama, K. and Hirata, K.: Plant Science 41, 179-183 (1985)) supplemented with acetosyringone, and the OD₆₀₀ was adjusted to 0.18 to 0.2. They were shake -cultured in the dark at 25° C for 1 hour.

The rice calli cultured in the above manner were placed in a sterilized tea strainer, and the above cultured A. *tumefaciens* in the form of a suspension was added. The tea strainer was shaken for 3 minutes with occasional rooking for securing immersion of the whole calli, then the tea strainer and the contents were together placed on a paper towel, and the excessive bacterial culture fluid was removed. The calli were placed on co-culture medium and cultured in the dark at 25°C for 3 days.

The co-cultured calli were then collected in a tea strainer, immersed in sterilized water supplemented with 500 mg/l of claforan, the tea strainer was shaken to wash away A. *tumefaciens*, the tea strainer and the contents were then together placed on a sterilized paper towel, and the water was removed. The same procedure was repeated four times in all. The calli were placed on selection medium and cultured in the dark at 28°C. Three to four weeks later, calli were randomly selected from among a large number of calli, a portion of each selected callus was taken and placed in a GUS staining solution (0.75 mM X-Gulo (5-bromo-4-chloro-3-indolyl-β-D-glucuronic acid), 0.5 mM potassium ferrioyanide, 0.5 mM potassium ferrocyanide, 0.3% Triton X-100, 20% methanol, 50 mM phosphate buffer (pH 7.0)), and the reaction was allowed to proceed overnight at 37° C for detecting GUS activity. The callus stained blue and thus showing GUS activity is a transgenic, transformed rice callus. The results thus obtained are shown in Table 4.

**Table 4**

| Results of rice callus staining for GUS | | | |
|---|---|---|---|
| | 35S | IS1 | IS2 |
| Calluses stained | 27 | 38 | 44 |
| Total number of calluses | 275 | 244 | 228 |
| Percentage of transformant calluses (%) | 9.8 | 15.6 | 19.3 |

As is evident from the results shown in Table 4, the transformation efficiency was found increased by introduction of the constructs IS1 and IS2 to about 1.5 times and about 2 times, respectively, as compared with the control (35S).

The above-mentioned Examples 1 to 6 showed that the regeneration efficiency (transformation efficiency) is increased by using the following transcriptional activation elements (IS1, IS2, IS12, MU3). The following three possibilities can be considered as the reasons:
(1) The possibility of the efficiency of introduction of Ti plasmid into plant cells being increased;
(2) The possibility of the efficiency of regeneration from cells being increased as the result of an increase in nos promoter activity owing to the IS1 and/or IS2 element and consequent promotion of the transcription of the nptII gene, which leads to production of the gene product in an increased amount, and, hence, increase in number of cells capable of growing on the kanamycin-containing medium used for selection; and
(3) The possibility of the IS1 and/or IS2 element activating the gene region in the vicinity or marginal region thereof or preventing said gene region from being inactivated.

The gene introduction by means of Ti plasmid does not lead to insertion at a determined site on the plant chromosome but is indefinite as to the site of insertion. Therefore, it is accidental whether the gene in question is inserted in an active site determined by the structure of the chromosome or in a cryptic site in the vicinity of which a gene has been inactivated by methylation of the genomic DNA or by some other cause. It is thought that if a transgene is introduced in a cryptic site, it is influenced by the "field" of the chromosome, so that the transgene is also inactivated.

In the constructs used in the above examples, the transcriptional activation elements (IS1 or/and IS2) are found inserted on the terminator side of the nptII gene (kanamycin resistance gene), namely on the opposite side of the nos promoter of the kanamycin resistance gene. Therefore, it is impossible that these elements cis act on the promoter of the kanamycin resistance gene.

However, the above examples gave the results showing that even in the circumstances in which the transcriptional activation element of the present invention is found inserted in a position such that it cannot directly act on the nos promoter, the number of kanamycin resistant cells (transformant tobacco calli) increases in the case of cultured tobacco cells, in particular that even when the kanamycin concentration in medium is as high as 300 mM, the number of kanamycin resistant calli increases (Example 3 (1)), and further that the efficiency of regeneration of kanamycin resistant plants is increased by introducing the constructs mentioned above into plant cells of various plant species (Examples 4 to 6). These results indicate that the IS1 or/and IS2 elements acted on the kanamycin resistance gene occurring in the vicinity of said elements, not in the mode of directly causing ois activation of the nos promoter, and as a result, the number of cells retaining (includes both the senses of activation and prevention from being inactivated) the activity of said kanamycin resistance gene. Thus, this Indicates the possibility that, unlike the conventional transcriptional activation elements (factors) occurring as enhancers in the vicinity of the promoter of a specific gene and activating the transcription by cis acting on said promoter, the transcriptional activation element of the present invention is to activate (or prevent from inactivating), when it is inserted into a genomic gene, a single gene group or a plurality of gene groups in the vicinity or marginal region of the site of insertion thereof (irrespective of location and direction of the gene promoter on which it acts) and thus promoting the transcription activity, namely acting by the mechanisms mentioned above under (3).

In the constructs used in the above examples, the IS1 or/and IS2 elements were inserted on the 35S promoter side of the GUS gene. The cultured tobacco cells resulting from insertion of said construct showed increased GUS activity in Example 3 (2) indicated clearly that the transcriptional activation elements of the invention act also on the 35S promoter occurring in the downstream vicinity thereof to increase the transcription activity of the GUS gene. This result supports the judgment mentioned above and indicates that the present transcriptional activation elements show the actions mentioned above under (3), namely that "the IS1 or/and IS2 elements activate the neighboring gene region including them or prevent said gene region from being inactivated".

In the above example, it was also shown that, not only with cultured cells (Example 3) but also with plant tissues, the efficiency of regeneration of tobacco plant shoots is indeed increased in the tobacco leaf disk experiment (Example 4), the efficiency of formation of embryogenic callus serving as bases for plant regeneration from carrot hypocotyls is increased (Example 5), and the efficiency of formation of callus serving as bases for rice plant regeneration is increased (Example 6), by using the transcriptional activation elements of the invention. These results indicate that the transcriptional activation elements of the invention act on those plant cells becoming incapable of plant regeneration or callus formation as a result of a foreign gene introduced into the genome of plants in question undergoing gene silencing (inactivation) by the position effect, so as to increase the efficiency of plant regeneration or formation, hence are practically very useful.

In view of the recent finding that MITES, like MARs, bind to nuclear matrices (Tikhonov et al. , Plant Cell 12: 249-264 (2000)), it is considered that MITEs intranuolearly play a role similar to that of MARS. Based on this, it can be expected that the transcriptional activation elements of the invention, which are MITEs, can be used singly or in combination with such elements as MARs in producing genetically modified plants to further increase the efficiency of plant regeneration or formation.

### INDUSTRIAL APPLICABILITY

The most important problem to be overcome in producing genetically modified plants is the phenomenon of gene silencing which causes expression inactivation of foreign genes. For avoiding the gene silencing phenomenon in developing genetically modified plants, it is necessary to select, from among a large number of plant individuals, those plant individuals with the foreign gene in question inserted at such a site as causing gene silencing as least as possible.

The present invention provides novel, plant-derived MITE-like elements, and it is highly possible that said MITE-like elements, when inserted in a plant genome, cause changes in genomic structure and, based on this, contribute to changes in dynamics of the genomic structure, for example facilitating the unwinding of genomic DNA or causing changes in nucleosome structure, by mechanisms quite different from the mechanisms of action of the conventional enhancer elements.

Therefore, by utilizing this characteristic feature of the MITE-like elements of the invention, it will become possible to regulate the expression of a gene occurring in the vicinity thereof by techniques different from the prior art ones. In other words, with the MITE-like elements of the invention which have the above characteristic feature, it will be possible to increase or activate the reduced expression ability of a foreign gene introduced by the transgenic technology. In this respect, the MITE-like elements of the invention are useful in constructing a transgene expression cassette and plasmids containing said cassette in the production of genetically modified living organisms and is further useful in stably producing genetically modified organisms capable of expressing a transgene.

The transcriptional activation elements of the invention which contain a transposable element such as one of the MITE-like elements mentioned above (preferably the IS2 element) have an activity in suppressing and dissolving the phenomenon of inactivation of gene expression (gene silencing phenomenon) due to the position effect in gene transfer in plants. Therefore, it is expected that by using the transcriptional activation elements of the invention singly or in combination with other elements participating in nuclear DNA structuring, for example MARs (matrix attachment regions), it will become possible to have the gene stably expressed to thereby markedly reduce the number of screening procedures generally performed after gene transfer and the number of recombinant plants to be sowed and grown.

Such plants large in genomic size as lily, chrysanthemum and wheat have large cryptic sites within the genome and, therefore, a foreign gene introduced is mostly inserted in cryptic sites. For such plants, it is thus very difficult and practically impossible in the prior art to produce recombinant plants. On the contrary, with the transcriptional activation elements of the invention, it is possible to significantly inhibit a foreign gene introduced onto a plant genome from undergoing silencing and therefore it is expected that said elements can make it possible to efficiently introduce foreign genes into those plant species the gene recombination of which has been regarded as difficult, as mentioned above, and produce recombinant plants.

The transcriptional activation elements of the invention are also considered to be elements capable of activating (inclusive of transcriptional activation) genes occurring in the vicinity or marginal region of the gene region in which they are found inserted. Therefore, the transcriptional activation elements of the invention are expected to not only make it possible to put the transgenic technology to practical use even in those plants in which the production of genetically modified plant bodies is difficult because of frequent occurrence of silencing due to the large genome size, as mentioned above, but also make it possible, even in the genetic engineering of plant species such as soybean, corn, potato and tomato, already put to practical use, to increase the efficiency of gene transfer and, at the same time, increase the activity of transcription of genes for useful characters, such as herbicide resistance genes and insecticidal protein genes, by using these transcriptional activation elements through insertion at a site upstream of the promoter of such a gene for a useful character, or at a site in the vicinity thereof. They are further expected to make it possible to produce genetically modified plants with higher productivity and higher quality as compared with the conventional methods of producing genetically modified plants.

## Claims

1. A MITE-like element consisting of the following DNA (a) or (b):
(a) a DNA having a nucleotide sequence shown under SEQ ID NO:1
(b) a DNA capable of hybridizing with a DNA having a complementary sequence to the above nucleotide sequence (a) under stringent conditions and capable of causing duplication of the target sequence: (A)nG(A)n [n being an integer of not less than 1] at the site of insertion thereof in a genomic gene.

2. A transcriptional activation element **characterized by** containing at least one MITE-like element of claim 1 as a transposable element.

3. A transcriptional activation element as claimed in Claim 2, wherein the transposable element comprises at least one MITE-like element consisting of the following DNA
(a) or (b):
(a) a DNA having the nucleotide sequence shown under SEQ ID No: 1:
(b) a DNA capable of hybridizing with a DNA having a complementary sequence to the above nucleotide sequence (a) under stringent conditions and capable of causing duplication of (A)nG(A)n [n being an integer of not less than 1] at the site of insertion thereof in a genomic gene.

4. A transcriptional activation element as claimed in Claim 2, wherein the transposable element is a tandem coupling product from a MITE-like element consisting of the following DNA (a) or (b):
(a) a DNA having the nucleotide sequence shown under SEQ ID NO:1:
(b) a DNA capable of hybridizing with a DNA having a complementary sequence to the above nucleotide sequence (a) under stringent conditions and capable of causing duplication of (A) nG (A) n [n being an integer of not less than 1] at the site of insertion thereof in a genomic gene,
and a MITE-like element consisting of the following DNA (c) or (d):
(c) a DNA having the nucleotide sequence shown under SEQ ID NO: 2
(d) a DNA capable of hybridizing with a DNA having a complementary sequence to the above nucleotide sequence (c) under stringent conditions and capable of causing duplication of TA at the site of insertion thereof in a genomic gene.

5. A transcriptional activation element comprising a DNA having the nucleotide sequence shown under SEQ ID NO:3.

6. A transcriptional activation element comprising a DNA having the nucleotide sequence shown under SEQ ID NO:14.

7. A transgene expression cassette which comprises the transcriptional activation element of any of Claims 2 to 6, and a DNA sequence operatively joined to said element.

8. A transgene expression cassette as claimed in Claim 7, wherein the DNA sequence operatively joined to the transcriptional activation element comprises a promoter and/or a terminator.

9. A transgene expression cassette as claimed in Claim 8, which further comprises, as the DNA sequence operatively joined to the transcriptional activation element a desired transgene sequence to be expressed.

10. A plasmid containing the transcriptional activation element of any of Claims 2 to 6.

11. A plasmid containing the transgene expression cassette of any of Claims 7 to 9.

12. A transgenic plant which contains the transgene expression cassette of any of Claims 7 to 9.

13. A transgenic plant as claimed in Claim 12 which is corn, rice, wheat, lily, chrysanthemum, cotton, soybean, beet, potato or carica papaya.

## Patentansprüche

1. MITE-ähnliches Element, bestehend aus der folgenden DNA (a) oder (b):
(a) eine DNA mit einer unter SEQ ID Nr.:1 gezeigten Nukleotidsequenz,
(b) eine DNA, die befähigt ist, mit einer DNA, die eine komplementäre Sequenz zu der vorstehenden Nukleotidsequenz (a) aufweist, unter stringenten Bedingungen zu hybridisieren, und die befähigt ist, eine Verdopplung der Zielsequenz: (A)nG(A)n [wobei n eine ganze Zahl von nicht weniger als 1 ist] an deren Insertionsstelle in einem genomischen Gen zu bewirken.

2. Transkriptionelles Aktivierungselement, **dadurch gekennzeichnet, daß** es mindestens ein MITE-ähnliches Element nach Anspruch 1 als transponierbares Element enthält.

3. Transkriptionelles Aktivierungselement nach Anspruch 2, wobei das transponierbare Element mindestens ein MITE-ähnliches Element, bestehend aus der folgenden DNA (a) oder (b):
(a) eine DNA mit der unter SEQ ID Nr.:1 gezeigten Nukleotidsequenz,
(b) eine DNA, die befähigt ist, mit einer DNA, die eine komplementäre Sequenz zu der vorstehenden Nukleotidsequenz (a) aufweist, unter stringenten Bedingungen zu hybridisieren, und die befähigt ist, eine Verdopplung von (A)nG(A)n [wobei n eine ganze Zahl von nicht weniger als 1 ist] an dessen Insertionsstelle in einem genomischen Gen zu bewirken,
umfaßt.

4. Transkriptionelles Aktivierungselement nach Anspruch 2, wobei das transponierbare Element ein Tandem-Kopplungsprodukt eines MITE-ähnlichen Elements, bestehend aus der folgenden DNA (a) oder (b):
(a) eine DNA mit der unter SEQ ID Nr.:1 gezeigten Nukleotidsequenz,
(b) eine DNA, die befähigt ist, mit einer DNA, die eine komplementäre Sequenz zu der vorstehenden Nukleotidsequenz (a) aufweist, unter stringenten Bedingungen zu hybridisieren, und die befähigt ist, eine Verdopplung von (A)nG(A)n [wobei n eine ganze Zahl von nicht weniger als 1 ist] an dessen Insertionsstelle in einem genomischen Gen zu bewirken,
und eines MITE-ähnliches Elements, bestehend aus der folgenden DNA (c) oder (d):
(c) eine DNA mit der unter SEQ ID Nr.:2 gezeigten Nukleotidsequenz,
(d) eine DNA, die befähigt ist, mit einer DNA, die eine komplementäre Sequenz zu der vorstehenden Nukleotidsequenz (c) aufweist, unter stringenten Bedingungen zu hybridisieren, und die befähigt ist, eine Verdopplung von TA an dessen Insertionsstelle in einem genomischen Gen zu bewirken,
ist.

5. Transkriptionelles Aktivierungselement, umfassend eine DNA mit der unter SEQ ID Nr.: 3 gezeigten Nukleotidsequenz.

6. Transkriptionelles Aktivierungselement, umfassend eine DNA mit der unter SEQ ID Nr.: 14 gezeigten Nukleotidsequenz.

7. Transgene Expressionskassette, die das transkriptionelle Aktivierungselement nach einem der Ansprüche 2 bis 6 und eine DNA-Sequenz, die operativ an das Element gebunden ist, umfaßt.

8. Transgene Expressionskassette nach Anspruch 7, wobei die DNA-Sequenz, die operativ an das transkriptionelle Aktivierungselement gebunden ist, eine Promotor und/oder einen Terminator umfaßt.

9. Transgene Expressionskassette nach Anspruch 8, die weiter eine gewünschte transgene zu exprimierende Sequenz als die DNA-Sequenz, die operativ an das transkriptionelle Aktivierungselement gebunden ist, umfaßt.

10. Plasmid, enthaltend das transkriptionelle Aktivierungselement nach einem der Ansprüche 2 bis 6.

11. Plasmid, enthaltend die transgene Expressionskassette nach einem der Ansprüche 7 bis 9.

12. Transgene Pflanze, welche die transgene Expressionskassette nach einem der Ansprüche 7 bis 9 enthält.

13. Transgene Pflanze nach Anspruch 12, die Mais, Reis, Weizen, eine Lilie, eine Chrysantheme, Baumwolle, eine Sojabohne, eine Rübe, eine Kartoffel oder ein Melonenbaum ist.

## Revendications

1. Elément de type MITE constitué par l'ADN (a) ou (b) suivant :
(a) un ADN ayant une séquence nucléotidique illustrée par SEQ ID NO : 1
(b) un ADN capable de s'hybrider à un ADN ayant une séquence complémentaire de la séquence nucléotidique (a) ci-dessus dans des conditions stringentes et capable de provoquer la duplication de la séquence cible : (A)nG(A)n [n étant un nombre entier qui n'est pas inférieur à 1] au niveau du site d'insertion de celui-ci dans un gène génomique.

2. Élément d'activation de la transcription **caractérisé en ce qu'**il contient au moins un élément de type MITE selon la revendication 1 en tant qu'élément transposable.

3. Elément d'activation de la transcription selon la revendication 2, dans lequel l'élément transposable comprend au moins un élément de type MITE constitué par l'ADN (a) ou (b) suivant:
(a) un ADN ayant une séquence nucléotidique illustrée par SEQ ID NO : 1
(b) un ADN capable de s'hybrider à un ADN ayant une séquence complémentaire de la séquence nucléotidique (a) ci-dessus dans des conditions stringentes et capable de provoquer la duplication de (A)nG(A)n [n étant un nombre entier qui n'est pas inférieur à 1] au niveau du site d'insertion de celui-ci dans un gène génomique.

4. Elément d'activation de la transcription selon la revendication 2, dans lequel l'élément transposable est un produit de couplage en tandem issu d'un élément de type MITE constitué par l'ADN (a) ou (b) suivant :
(a) un ADN ayant une séquence nucléotidique illustrée par SEQ ID NO : 1
(b) un ADN capable de s'hybrider à un ADN ayant une séquence complémentaire de la séquence nucléotidique (a) ci-dessus dans des conditions stringentes et capable de provoquer la duplication de (A)nG(A)n [n étant un nombre entier qui n'est pas inférieur à 1] au niveau du site d'insertion de celui-ci dans un gène génomique,
et d'un élément de type MITE constitué par l'ADN (c) ou (d) suivant :
(c) un ADN ayant la séquence nucléotidique illustrée par SEQ ID NO : 2
(d) un ADN capable de s'hybrider à un ADN ayant une séquence complémentaire de la séquence nucléotidique (c) ci-dessus dans des conditions stringentes et capable de provoquer la duplication de TA au niveau du site d'insertion de celui-ci dans un gène génomique.

5. Elément d'activation de la transcription comprenant un ADN ayant la séquence nucléotidique illustrée par SEQ ID NO:3.

6. Elément d'activation de la transcription comprenant un ADN ayant la séquence nucléotidique illustrée par SEQ ID NO : 14.

7. Cassette d'expression de transgène qui comprend l'élément d'activation de la transcription selon l'une quelconque des revendications 2 à 6, et une séquence d'ADN liée de manière fonctionnelle au dit élément.

8. Cassette d'expression de transgène selon la revendication 7, dans laquelle la séquence d'ADN liée de manière fonctionnelle à l'élément d'activation de la transcription comprend un promoteur et/ou un terminateur.

9. Cassette d'expression de transgène selon la revendication 8, qui comprend en outre, en tant que séquence d'ADN liée de manière fonctionnelle à l'élément d'activation de la transcription, une séquence de transgène souhaitée à exprimer.

10. Plasmide contenant l'élément d'activation de la transcription selon l'une quelconque des revendications 2 à 6.

11. Plasmide contenant la cassette d'expression de transgène selon l'une quelconque des revendications 7 à 9.

12. Plante transgénique qui contient la cassette d'expression de transgène selon l'une quelconque des revendications 7 à 9.

13. Plante transgénique selon la revendication 12 qui est le maïs, le riz, le blé, le lis, le chrysanthème, le coton, le soja, la betterave, la pomme de terre ou la papaye.


### SEQUENCE LISTING

<110> OZEKI, Yoshihiro
<110> SAN-EI GEN F.F.I.,INC.,
<120> NOVEL MINIATURE INVERTED-REPEAT TRANSPOSABLE ELEMENTS (MITEs)-LIKE ELEMENT AND TRANSCRIPTIONAL ACTIVATION ELEMENT
<130> P00-14
<150> JP 1999/206316
<151> 1999-07-21
<150> JP 1999/206320
<151> 1999-07-21
<150> JP 2000/175825
<151> 2000-06-12
<160> 14
<210> 1
<211> 769
<212> DNA
<213> Carrot (Daucus carota L.cv.Kurodagosun)
<400> 1
   gggatctttt taaaaatacc catctgtaaa attatttttt taaaaatact accatctttt   60
   tcattgtttt taaaaatacc ttttcataaa tttttttttt caaaaatacg atttgcaact  120
   tttgcaacct catttgcaac cttgggcggc gcagccgtaa aagttgccag tgaggttgca  180
   aaagttgcaa atgagtttgt aaaagttgca aatgaggttg caaaagttgc aaataaaaat  240
   ggaaagttgc aacagttgca actgcaattg caactagttc aactgaaaac tgtaagttgc  300
   aaaagttgca aatgaggttg caactaaatg caactgaaaa ctgtaagtaa caacagatgt  360
   atggtgtgcc cctggcgggg ccgttagatt acaatagaat caactgaatg caatcatatg  420
   caactgaata caactatatg caatcatata tgcaattaca aatcctgatt tcaagttcca  480
   gttttcgaat gtcattttcg aaatcgatat atatatatat atatatatat cgatttcgaa  540
   aatgacattc gaaaactgga acttgaaatc aggaattcag ctgcatatga agttgcaaaa  600
   gaggttgcaa cacggctggc gccgcctgta gttgcaaatg aggttgcaaa agttgcaaac  660
   agtatttttg aaaaaaagat tttatgaaaa ggtattttta aaaataattc tggaaggtag  720
   tatttttgaa aacaataaaa gaaaaggtag gtagttttgt agatttccc              769
<210> 2
<211> 299
<212> DNA
<213> Carrot (Daucus carota L.cv.Kurodagosun)
<400> 2
   ctccctacgt cccattttat gtgacctcat tttctttttg ggacgtctca aaaaaaataa 60
   cctagaatac ttactatttt ttaacactat ttttcactat tacacccacc aactctatat 120
   tttatactat tttattatta aataaacact attacaccca ctacttttct ccactatctc 180
   aaatctatta ttaaatattg ataggtccac cactttaccc acttttcaac tacatttact 240
   acatttttct taatctccgt gaaagtcaaa ctcattcaca taaaatggga cagagggag 299
<210> 3
<211> 1192
<212> DNA
<213> Carrot (Daucus carota L.cv.Kurodagosun)
<400> 3
   ggtaccgggc cccccctcga ggtcactccc tacgtcccat tttatgtgac ctcattttct 60
   ttttgggacg tctcaaaaaa aataacctag aatacttact attttttaac actatttttc  120
   actattacac ccaccaactc tatattttat actattttat tattaaataa acactattac  180
   acccactact tttctccact atctcaaatc tattattaaa tattgatagg tccaccactt  240
   tacccacttt tcaactacat ttactacatt tttcttaatc tccgtgaaag tcaaactcat  300
   tcacataaaa tgggacagag ggagtaatta ttaattttaa tagacggtat cgataagctt  360
   atcgataccg tctcagattc gcaaacataa aaagaaaagg gatcttttta aaaataccca  420
   tctgtaaaat tattttttta aaaatactac catctttttc attgttttta aaaatacctt  480
   ttcataaatt ttttttttca aaaatacgat ttgcaacttt tgcaacctca tttgcaacct  540
   tgggcggcgc agccgtaaaa gttgccagtg aggttgcaaa agttgcaaat gagtttgtaa  600
   aagttgcaaa tgaggttgca aaagttgcaa ataaaaatgg aaagttgcaa cagttgcaac  660
   tgcaattgca actagttcaa ctgaaaactg taagttgcaa aagttgcaaa tgaggttgca  720
   actaaatgca actgaaaact gtaagtaaca acagatgtat ggtgtgcccc tggcggggcc  780
   gttagattac aatagaatca actgaatgca atcatatgca actgaataca actatatgca  840
   atcatatatg caattacaaa tcctgatttc aagttccagt tttcgaatgt cattttcgaa  900
   atcgatatat atatatatat atatatatcg atttcgaaaa tgacattcga aaactggaac  960
   ttgaaatcag gaattcagct gcatatgaag ttgcaaaaga ggttgcaaca cggctggcgc 1020
   cgcctgtagt tgcaaatgag gttgcaaaag ttgcaaacag tatttttgaa aaaaagattt 1080
   tatgaaaagg tatttttaaa aataattctg gaaggtagta tttttgaaaa caataaaaga 1140
   aaaggtaggt agttttgtag atttcccaga cctcgagggg gggcccggta cc         1192
<210> 4
<211> 8
<212> DNA
<213> Carrot (Daucus carota L.cv.Kurodagosun)
<400> 4
   gttgcaaa 8
<210> 5
<211> 8
<212> DNA
<213> Carrot (Daucus carota L.cv.Kurodagosun)
<400> 5
   gttgcaac 8
<210> 6
<211> 8
<212> DNA
<213> Carrot (Daucus carota L.cv.Kurodagosun)
<400> 6
   tttgcaaa 8
<210> 7
<211> 8
<212> DNA
<213> Carrot (Daucus carota L.cv.Kurodagosun)
<400> 7
   tttgcaac 8
<210> 8
<211> 7
<212> DNA
<213> Carrot (Daucus carota L.cv.Kurodagosun)
<400> 8
   tatgcaa 7
<210> 9
<211> 7
<212> DNA
<213> Carrot (Daucus carota L.cv.Kurodagosun)
<400> 9
   aatgcaa 7
<210> 10
<211> 158
<212> DNA
<213> Carrot (Daucus carota L.cv.Kurodagosun)
<400> 10
   gggatctttt taaaaatacc catctgtaaa attatttttt taaaaatact accatctttt 60
   tcattgtttt taaaaatacc ttttcataaa tttttttttt caaaaatacg atttgcaact 120
   tttgcaacct catttgcaac cttgggcggc gcagccgt 158
<210> 11
<211> 158
<212> DNA
<213> Carrot (Daucus carota L.cv.Kurodagosun)
<400> 11
   acggctggcg ccgcctgtag ttgcaaatga ggttgcaaaa gttgcaaaca gtatttttga 60
   aaaaaagatt ttatgaaaag gtatttttaa aaataattct ggaaggtagt atttttgaaa 120
   acaataaaag aaaaggtagg tagttttgta gatttccc 158
<210> 12
<211> 32
<212> DNA
<213> Carrot (Daucus carota L.cv.Kurodagosun)
<400> 12
   ctccctacgt cccattttat gtgacctcat tt 32
<210> 13
<211> 32
<212> DNA
<213> Carrot (Daucus carota L.cv.Kurodagosun)
<400> 13
   aaactcattc acataaaatg ggacagaggg ag 32
<210> 14
<211> 1553
<212> DNA
<213> Carrot (Daucus carota L.cv.Kurodagosun)
<400> 14
   ggtaccgggc cccccctcga ggtcactccc tacgtcccat tttatgtgac ctcattttct 60
   ttttgggacg tctcaaaaaa aataacctag aatacttact attttttaac actatttttc  120
   actattacac ccaccaactc tatattttat actattttat tattaaataa acactattac  180
   acccactact tttctccact atctcaaatc tattattaaa tattgatagg tccaccactt  240
   tacccacttt tcaactacat ttactacatt tttcttaatc tccgtgaaag tcaaactcat  300
   tcacataaaa tgggacagag ggagtaatta ttaattttaa taaattatat gtgattttga  360
   ttatgtgtgg attcttgata aaatatcaca gagttgatat aaattctaaa gatttaacca  420
   caatgtttca aaatctcata gattttagta ggattcaaaa aatttaaaat acgttcaaaa  480
   atctcataaa attcatcatt ttattaaatc caaaaaaatc cagtaatatt tgacaatcag  540
   attttaatga attttaaatt gaacaatctc agttgaatac catgagattt taaaatataa  600
   tttaaaattc taattgaata ccaccagatt ttgtaaaata atttaaaatt ttaattgaat  660
   attcaagatt ttaatatatt ttaaataatc tcgatctgaa ttacaaaaaa tgcttaaaat  720
   ctgaatccca tacgtcctct cagattcgca aacataaaaa gaaaagggat ctttttaaaa  780
   atacccatct gtaaaattat ttttttaaaa atactaccat ctttttcatt gtttttaaaa  840
   ataccttttc ataaattttt tttttcaaaa atacgatttg caacttttgc aacctcattt  900
   gcaaccttgg gcggcgcagc cgtaaaagtt gccagtgagg ttgcaaaagt tgcaaatgag  960
   tttgtaaaag ttgcaaatga ggttgcaaaa gttgcaaata aaaatggaaa gttgcaacag 1020
   ttgcaactgc aattgcaact agttcaactg aaaactgtaa gttgcaaaag ttgcaaatga 1080
   ggttgcaact aaatgcaact gaaaactgta agtaacaaca gatgtatggt gtgcccctgg 1140
   cggggccgtt agattacaat agaatcaact gaatgcaatc atatgcaact gaatacaact 1200
   atatgcaatc atatatgcaa ttacaaatcc tgatttcaag ttccagtttt cgaatgtcat 1260
   tttcgaaatc gatatatata tatatatata tatatcgatt tcgaaaatga cattcgaaaa 1320
   ctggaacttg aaatcaggaa ttcagctgca tatgaagttg caaaagaggt tgcaacacgg 1380
   ctggcgccgc ctgtagttgc aaatgaggtt gcaaaagttg caaacagtat tttgaaaaaa 1440
   aagattttat gaaaaggtat ttttaaaaat aattctggaa ggtagtattt ttgaaaacaa 1500
   taaaagaaaa ggtaggtagt tttgtagatt tcccagacgg tatcgataag ctt        1553